# EUROPEAN PATENT SPECIFICATION

(11) **EP 3 704 243 B1**
(45) Date of publication and mention of the grant of the patent: **30.08.2023**
(21) Application number: 18800295.0
(22) Date of filing: 01.11.2018
(51) Int. Cl.: C12N 15/10, C12R 1/07, A61K 35/742, C12N 15/75

(54) **MODIFIED BACTERIAL SPORES**
MODIFIZIERTE BAKTERIELLE SPOREN
SPORES BACTÉRIENNES MODIFIÉES

(30) Priority: 02.11.2017 GB 201718133
(43) Date of publication of application: 09.09.2020
(73) Proprietor: SporeGen Limited, London NW1 0NH (GB)
(72) Inventor: CUTTING, Simon, London NW1 0NH (GB)
(74) Representative: Hutter, Anton
(86) International application number: PCT/GB2018/053178
(87) International publication number: WO 2019/086887

(56) References cited:
- WO-A1-2010/034844
- A. SEKOWSKA ET AL: "Identification of yrrU as the Methylthioadenosine Nucleosidase Gene in Bacillus subtilis", DNA RESEARCH., vol. 6, no. 5, 1 January 1999 (1999-01-01) , pages 255-264, XP055531986, JP ISSN: 1340-2838, DOI: 10.1093/dnares/6.5.255
- NORBERTO VILLEGAS-NEGRETE ET AL: "Implementation of a loss-of-function system to determine growth and stress-associated mutagenesis in Bacillus subtilis", PLOS ONE, vol. 12, no. 7, 11 July 2017 (2017-07-11), page e0179625, XP055532608, DOI: 10.1371/journal.pone.0179625
- EDWARD M. RUBIN ET AL: "Expression of thymidylate synthetase activity in Bacillus subtilis upon integration of a cloned gene from Escherichia coli", GENE., vol. 10, no. 3, 1 August 1980 (1980-08-01) , pages 227-235, XP055533107, NL ISSN: 0378-1119, DOI: 10.1016/0378-1119(80)90052-9
- HOSSEINI SIAMAND ET AL: "Biological Containment of Genetically Modified Bacillus subtilis", APPLIED AND ENVIRONMENTAL MICROBIOLOGY, vol. 84, no. 3, February 2018 (2018-02), XP002787358,

## Description

The present invention relates to modified bacterial spores, and particularly, although not exclusively, to modified bacterial spores which comprise two or more heterologous genes, but without the introduction of antibiotic resistance genes. The invention extends to methods for producing such modified bacterial spores, vectors and kits for introducing heterologous genes in a spore-forming bacterium.

*Bacillus* endospores, or spores, are dormant entities with well-known resistance properties including heat stability, exposure to noxious chemicals and desiccation (1). As such, spores offer a number of applications. First and foremost has been their use as mucosal vaccines where antigens can be displayed on the spore surface by fusion to a coat protein anchor (2). Immunization of recombinant spores by a mucosal route (oral, sub-lingual or nasal) has shown promising results and in some cases levels of protection that could be efficacious in humans or animals (3-5). In the case of the latter, the ability to incorporate a vaccine in feed is particularly attractive and for some animals, arguably, the only way to vaccinate. For example, vaccinating farmed shrimp against viral pathogens, for example, White Spot Syndrome Virus (WSSV) where recombinant spores expressing the WSSV VP26 and VP28 envelope proteins have been shown to confer protection in shrimps (6-9).

A number of *Bacillus* species are considered `food grade', that is, safe for human consumption and are designated as QPS (Qualified Presumption of Safety as defined by EFSA, European Food Safety Authority) (10). In the USA, some strains carry GRAS (Generally Recognized As Safe as defined by the Food and Drug Administration) status. QPS and GRAS status has supported the use of a number of *Bacillus* strains, including those of *B. subtilis,* as probiotics in both human and animal feeds (11). The concept of recombinant probiotics (12) is conceptually, a logical next step forward in exploiting the beneficial properties of *Bacillus* (13).

In addition to vaccines, spores have been shown to facilitate expression of enzymes on the spore surface, for example the animal feed enzyme phytase (14). The ability to use probiotic bacteria that deliver enzymes negates the need to purify enzymes and could offer significant advantages to industry. Finally, streptavidin has been expressed on *B*. *subtilis* spores enabling monoclonal antibodies to be conjugated to the spore surface and the targeting of spores loaded with anti-cancer drugs to cancer cells (15).

A. Sekowaska et al., DNA Research, vol. 6, no. 5 (1999-01-01), pages 255-264, discloses a method of selecting *Bacillus subtilis* grown in the presence of trimethoprim. Norberto Villegas-Negrete et al., PLOS One, vol. 12, no. 7 (2017-07-11), page 00179625, teaches a method of disrupting the *thyA* and *thyB* loci using antibiotic resistance markers, neomycinR and erythromycin. WO 2010/034844 A1 discloses the inactivation of the *thyA* gene in Clostridium species. Edward M. Rubin et al., Gene, vol. 10, no.3 (1980-08-01), pages 227-235, discloses a method in which a wild type thymidylate synthetase gene is added to the genome of a strain carrying a thymine mutation, using plasmid DNA.

Despite the potential utility of *Bacillus* spores for industrial applications, there remain a number of obstacles regarding the deliberate release of genetically modified *Bacillus.* First, is the use of antibiotic resistance (Ab^{R}) genes used in the genetic engineering of stable recombinant strains. Regarding Ab^{R} the majority of procedures requiring insertion of heterologous DNA into *B. subtilis* require ectopic insertion using an Ab^{R} gene for positive selection (16). Plasmid vectors carrying chloramphenicol or erythromycin resistance genes are typically used for ectopic insertion at a genetic locus that is redundant for cell growth, typically the alpha amylase (amyE) or threonine C (*thrC*) genes (17). The potential risk of Ab^{R} gene transfer following release is recognised and at least two systems in *B. subtilis* have now been described that enable insertion of heterologous genes without introduction of an Ab^{R} gene (18, 19).

The second, and most challenging, hurdle is the ultimate fate of recombinant spores once released, since spores have been shown to survive for millions of years as viable entities (20). The soil is normally enriched with dormant spores (21) and their intrinsic robustness makes it difficult to argue that they would not persist after deliberate release. One approach might be to construct germination deficient spores but, at best, the germination rate can be reduced to 0.0015% (22) which is unlikely to satisfy regulatory authorities.

Therefore, there is a need for improved methods of introducing heterologous genes into spore-forming bacteria without the introduction of antibiotic resistance genes.

The inventors have adopted the approach of thymine starvation resulting in a so-called 'thymine-less death' (23-25). The majority of prokaryotes are known to carry a single thymidylate synthase enzyme that is encoded by the *thyA* gene and prokaryotes carrying a mutated thymidylate synthase gene are unable to grow in low concentrations of thymidine or thymine resulting in cell death. Thymine starvation therefore is different from the biostatic effects commonly found with deprivation of other nutrients. Thymine-less death has been documented for *B. subtilis* (26) and the bacterium is unique in that it carries two thymidylate synthase enzymes encoded by the thyA and *thyB* genes (27).

However, the concept of thymine-less death, despite being known since 1954, has never been successfully applied to spore-forming bacteria for the purposes of introducing heterologous genes without the introduction of antibiotic resistance genes.

The inventors have successfully adopted the principles of a 'thymine-less death' to exploit spore-forming bacteria, such as *Bacillus,* for the introduction of heterologous genes without the introduction of antibiotic resistance genes. As recombinant spores, should they germinate, they will die immediately and fail to survive or proliferate (i.e., grow) in the environment due to the lack of (or only low concentrations of) thymine or thymidine. The approach developed is particularly novel since it is a two-step gene integration process enabling positive selection on successively higher levels of trimethoprim, but without the introduction of any antibiotic resistance genes.

The invention is as defined in the claims.

In a first aspect of the invention, there is provided a process for introducing at least two heterologous genes into a *Bacillus Spp* and rendering the *Bacillus Spp* unable to proliferate in the absence of thymine or thymidine, the process comprising: (a) (i) introducing a heterologous gene into the *thyA* gene and the *thyB* gene in a *Bacillus Spp;* and (ii) growing said *Bacillus Spp* in the presence of trimethoprim; or (b) (i) introducing a heterologous gene into either the *thyA* gene or the *thyB* gene in a *Bacillus Spp;* ii) growing said *Bacillus Spp* in the presence of trimethoprim; iii) introducing a heterologous gene into the other of the *thyA* gene or the *thyB* gene in said *Bacillus Spp.;* and (iv) growing said *Bacillus Spp.* in the presence of trimethoprim, wherein the process operates without the introduction of an antibiotic resistance gene.

In a second aspect of the invention, there is provided a bacterial spore obtained or obtainable by the process of the first aspect.

In a third aspect of the invention, there is provided a bacterial spore comprising at least two heterologous genes and unable to proliferate in the absence of thymine or thymidine, wherein the bacterial spore is produced by a process comprising: (i) introducing a heterologous gene into the *thyA* gene and the *thyB* gene in a *Bacillus Spp* ; (ii) growing said *Bacillus Spp* in the presence of trimethoprim; and (iii) forming a bacterial spore from said *Bacillus Spp,* wherein the bacterial spore does not comprise an antibiotic resistance gene.

In a fourth aspect of the invention, there is provided a vector for use in the process of the first aspect, wherein the vector comprises at least a portion of the 5' portion of a *Bacillus Spp. thyA* gene, a heterologous gene, and at least a portion of the 3' portion of a *Bacillus Spp. thyA* gene, and further comprising at least a portion of the 5' portion of a *Bacillus Spp. thyB* gene, a heterologous gene, and at least a portion of the 3' portion of a *Bacillus Spp. thyB* gene.

Various embodiments which are not part of the invention are included in the description, not all of which are encompassed by the claims.

Thus, according to a first example, there is provided a process for introducing at least one heterologous gene into a spore-forming bacterium and rendering the bacterium unable to proliferate in the absence of thymine or thymidine, the process comprising:
(i) introducing a heterologous gene into a thymidylate synthase gene within a spore-forming bacterium; and
(ii) growing said spore-forming bacterium in the presence of trimethoprim.

In a preferred embodiment, and advantageously, the process operates without the introduction of an antibiotic resistance gene, and therefore overcomes the problems discussed above.

In a preferred embodiment, the method further comprises:
(iii) forming a bacterial spore from said spore-forming bacterium.

It will be appreciated that the bacterial spore may or may not germinate, but if it does germinate, it is unable to grow out of that germinated spore and proliferate.

Advantageously, the inventor has devised an elegant way to inactivate all thymidylate synthases in a bacterium, for example in embodiments in which the bacterium carries two or more thy genes, or where the bacterium carries a *thyA* and a *thyB* gene (e.g., *Bacillus spp.),* or where the inactivation of *thyA* is unable to affect the growth of the bacterium under normal physiological conditions.

Hence, in a preferred embodiment, one or more heterologous genes is introduced into each thymidylate synthase gene present within the bacterium. For instance, in one embodiment, the spore-forming bacterium may be a *Bacillus Spp.* (which is unique in that it is known to have two thymidylate synthase genes, *thyA* and *thyB).* Preferably, a heterologous gene is introduced into each of the two thymidylate synthase genes, for example *thyA* and *thyB* of *Bacillus spp.* The *Bacillus spp.* bacterium may be *B. subtilis, B. cereus, B. pumilus, B. amyloliquefaeceans, B. coagulans, B. clausii, B. licheniformis or B. megaterium.* Most preferably, however, the *Bacillus spp.* bacterium is *B. subtilis.*

It should be appreciated that inactivation of *thyA* only in *Bacillus* will not induce a thymineless death, and will not damage or prevent growth under normal physiological conditions.

In another example, the spore-forming bacterium may be *Clostridium Spp.* (which is known to have one thymidylate synthase gene). Preferably, in this embodiment, a heterologous gene is introduced into the thymidylate synthase gene. The *Clostridium spp.* bacterium may be C. *difficile, C. perfringens, C. tetani,* C. *botulinum, C. acetobutylicum, C. cellulolyticum,* C. *novyi* or C. *thermocellum.* Most preferably, the *Clostridium spp.* bacterium is *Clostridium difficile.*

In preferred embodiments of the process, the spore-forming bacterium is present in a bacterial culture.

Step (ii) of the process may be performed using any suitable vessel, such as a Petri dish or glass culture flask, containing any growth medium as disclosed herein. The growth medium may comprise trimethoprim at a concentration of 0.001 µg/ml to 20 µg/ml, 0.01 µg/ml to 15 µg/ml, 0.1 µg/ml to 10 µg/ml, 1 µg/ml to 5 µg/ml, 2 µg/ml to 4 µg/ml, or preferably approximately 3 µg/ml.

In a further embodiment, during step (ii), the bacterium may be grown in a medium comprising yeast extract (YE) at a concentration lower than 2 mg/ml, or any concentration disclosed herein. In an embodiment, the medium contains no YE.

In an embodiment, during step (ii), the bacterium may be grown in a medium comprising thymine or thymidine at any concentration disclosed herein, for instance approximately 50 µg/ml.

In an embodiment, during step (ii), the bacterium may be grown in a medium comprising trimethoprim at any concentration disclosed herein, for instance approximately 3 µg/ml.

In an embodiment, during step (ii), the bacterium may be grown in a medium comprising casamino acids (CAA) at any concentration disclosed herein, for instance approximately 0.2% (w/v).

In an embodiment, during step (ii), the bacterium may be grown in a medium supplemented with at least one amino acid or any amino acid mixture as disclosed herein.

In an embodiment, cells carrying a gene insertion may be verified by the inability to grow unless supplemented with thymine or thymidine. A further or alternative verification is to amplify, by PCR, the presence of the chimeric genes from transformants.

In a most preferred embodiment, the invention provides a cloning system suitable for biological containment of genetically modified *Bacillus Spp.,* such as *B. subtilis.*

Hence, according to this embodiment of the invention, there is provided a process for introducing at least one heterologous gene into a *Bacillus Spp.* and rendering the bacterium unable to proliferate in the absence of thymine or thymidine, the process comprising:
(i) introducing a heterologous gene into the *thyA* gene and the *thyB* gene in a *Bacillus Spp.;* and
(ii) growing said *Bacillus Spp.* in the presence of trimethoprim.

In a preferred embodiment, the heterologous gene is introduced into one of the thy genes, and then subsequently into the other thy gene. Preferably, therefore, there is provided a process for introducing at least one heterologous gene into a *Bacillus Spp.* and rendering the bacterium unable to proliferate in the absence of thymine or thymidine, the process comprising:
(i) introducing a heterologous gene into either the *thyA* gene or the *thyB* gene in a *Bacillus Spp.;*
(ii) growing said *Bacillus Spp.* in the presence of trimethoprim;
(iii) introducing a heterologous gene into the other of the *thyA* gene or the *thyB* gene in said *Bacillus Spp.;* and
(iv) growing said *Bacillus Spp.* in the presence of trimethoprim.

In a preferred embodiment, the process further comprises:
(v) forming a bacterial spore from said *Bacillus Spp.*

It will be appreciated that the bacterial spore may or may not germinate, but if it does germinate, it is unable to grow out of that germinated spore and proliferate. In a preferred embodiment, the process operates without the introduction of an antibiotic resistance gene. In a preferred embodiment, step (i) is the introduction of a heterologous gene into the *thyA* gene in a *Bacillus Spp.* and step (iii) is the introduction of a heterologous gene into the *thyB* gene in a *Bacillus Spp.*

In a preferred embodiment, the *Bacillus Spp.* is *B. subtilis.* In preferred embodiments, the *Bacillus Spp.* or *B. subtilis* is present in a bacterial culture.

In one embodiment, the open reading frame of *thyA* may be encoded by the nucleic acid sequence provided herein as SEQ ID NO: 1, as follows:

ATGACGCAATTCGATAAACAATACAATTCAATTATAAAGGATATTATCAATAATGGAATCTCAGACGAAGAGTTTGAT
GTAAGAACCAAGTGGGACTCAGATGGAACACCGGCACATACTCTAAGTGTAATCAGTAAGCAAATGAGATTCGACAAC
TCAGAGGTTCCGATTTTAACGACAAAAAAGGTTGCCTGGAAAACAGCCATTAAAGAGTTGCTCTGGATTTGGCAGCTG
AAATCTAATGATGTTAATGATTTAAACATGATGGGCGTCCATATTTGGGATCAGTGGAAACAAGAAGACGGAACCATC
GGACATGCATATGGATTTCAGCTGGGGAAGAAAAACAGAAGTCTAAATGGAGAAAAAGTGGATCAGGTAGACTATCTT
CTTCATCAATTGAAGAACAATCCATCTTCACGCAGACACATTACAATGCTGTGGAATCCTGATGAATTAGACGCAATG
GCCTTAACGCCATGTGTATACGAGACACAATGGTACGTTAAACATGGGAAACTCCACCTTGAGGTAAGAGCACGGAGC
AATGATATGGCATTGGGAAATCCATTCAATGTATTCCAGTATAATGTGTTGCAGCGCATGATTGCTCAAGTGACTGGT
TATGAGCTTGGTGAATATATCTTTAACATTGGGGATTGCCATGTGTACACACGTCATATAGACAATTTGAAAATTCAA
ATGGAAAGAGAACAGTTTGAAGCACCTGAACTATGGATCAATCCTGAAGTGAAAGATTTTTATGACTTTACCATTGAT
GATTTCAAGTTAATCAACTATAAACATGGGGACAAGCTTTTATTTGAGGTAGCGGTTTAA
[SEQ ID NO: 1]

Preferably, therefore, the process comprises introducing a heterologous gene into a nucleic acid sequence substantially as set out in SEQ ID NO: 1, or a fragment or variant thereof, within *Bacillus Spp.* In an alternative embodiment, the processes may comprise introducing a heterologous gene into *Bacillus Spp.* not into SEQ ID NO: 1 or a fragment or variant thereof, but such that the expression of SEQ ID NO: 1 is disrupted or reduced.

In one embodiment, the open reading frame of *thyA* may be encoded by a nucleic acid sequence which encodes an amino acid sequence provided herein as SEQ ID NO: 18, as follows:

MTQFDKQYNSIIKDIINNGISDEEFDVRTKWDSDGTPAHTLSVISKQMRFDNSEVPILTTKKVAWKTAIKELLWIWQL
KSNDVNDLNMMGVHIWDQWKQEDGTIGHAYGFQLGKKNRSLNGEKVDQVDYLLHQLKNNPSSRRHITMLWNPDELDAM
ALTPCVYETQWYVKHGKLHLEVRARSNDMALGNPFNVFQYNVLQRMIAQVTGYELGEYIFNIGDCHVYTRHIDNLKIQ
MEREQFEAPELWINPEVKDFYDFTIDDFKLINYKHGDKLLFEVAV
[SEQ ID NO: 18

SEQ ID NO: 1 and 18 are examples from *B. subtilis,* and thus variants of these sequences include the relevant *thyA* genes from all *Bacillus Spp.* It is important to note that the invention is applicable to any *thyA* of a *Bacillus Spp.* and that SEQ ID NO: 1 and 18 are intended to be illustrative and not limiting.

In one embodiment, the open reading frame of *thyB* may be encoded by the nucleic acid sequence provided herein as SEQ ID NO: 2, as follows:

ATGAAACAGTATAAGGATTTCTGCAGACATGTTTTAGAGCATGGTGAGAAAAAGGGAGACCGGACTGGGACCGGAACA
ATCAGCACTTTCGGATATCAAATGAGATTTAATTTACGGGAAGGCTTTCCGATGCTCACCACTAAAAAACTCCACTTT
AAATCAATTGCGCATGAACTGCTGTGGTTCTTAAAAGGAGATACGAATGTACGCTATCTGCAGGAAAACGGAGTGCGA
ATCTGGAATGAGTGGGCTGATGAAAACGGTGAACTTGGACCTGTATATGGCTCCCAATGGCGTTCTTGGCGGGGAGCT
GATGGAGAAACCATTGATCAAATTTCCCGTCTTATTGAAGATATTAAAACAAATCCGAACTCCAGACGCTTAATCGTC
AGCGCCTGGAATGTTGGTGAAATTGATAAAATGGCGTTGCCGCCGTGCCATTGCCTGTTCCAATTCTATGTGTCTGAC
GGCAAGCTGTCCTGTCAGCTGTATCAGCGCTCTGCCGATGTTTTCTTAGGTGTGCCGTTTAATATTGCATCTTATGCC
CTCCTAACCATGATCATTGCTCATGTGACTGGGCTTGAACCGGGCGAGTTCATCCATACGTTTGGTGATGTTCATATT
TACCAAAATCATATTGAACAAGTCAATTTGCAGCTGGAAAGAGATGTTAGACCGCTTCCGCAGCTTCGTTTCGCCAGA
AAGGTTGATTCTATTTTTAACTTTGCATTTGAGGACTTTATCATCGAGGATTATGATCCGCATCCTCATATAAAAGGG
GCGGTCAGCGTATGA
[SEQ ID NO: 2]

Preferably, therefore, the process comprises introducing a heterologous gene into a nucleic acid sequence substantially as set out in SEQ ID NO: 2, or a fragment or variant thereof, within *Bacillus Spp.* The processes may comprise introducing a heterologous gene into *Bacillus Spp.* not into SEQ ID NO: 2 or a fragment or variant thereof, but such that the expression from SEQ ID NO: 2 is disrupted or reduced.

In one embodiment, the open reading frame of *thyB* may be encoded by a nucleic acid sequence which encodes an amino acid sequence provided herein as SEQ ID NO: 19, as follows:

MKQYKDFCRHVLEHGEKKGDRTGTGTISTFGYQMRFNLREGFPMLTTKKLHFKSIAHELLWFLKGDTNVRYLQENGVR
IWNEWADENGELGPVYGSQWRSWRGADGETIDQISRLIEDIKTNPNSRRLIVSAWNVGEIDKMALPPCHCLFQFYVSD
GKLSCQLYQRSADVFLGVPFNIASYALLTMIIAHVTGLEPGEFIHTFGDVHIYQNHIEQVNLQLERDVRPLPQLRFAR
KVDSIFNFAFEDFIIEDYDPHPHIKGAVSV
[SEQ ID NO: 19]

SEQ ID NO: 2 and 19 are examples from *B. subtilis,* and thus variants of these sequences include the relevant *thyB* genes from all *Bacillus Spp.* It is important to note that the invention is applicable to any *thyB* of a *Bacillus Spp.* and that SEQ ID NO: 2 and 19 are intended to be illustrative and not limiting.

In an embodiment, step (i) may be performed by the transformation of *Bacillus Spp.* with a plasmid, which may be linearised, which may comprise at least a portion of the left arm of a *Bacillus Spp. thyA* gene, a heterologous gene, and at least a portion of the right arm of a *Bacillus Spp. thyA* gene. Step (i) may be performed with any vector as disclosed herein. Transformation may be carried out using electroporation.

Step (ii) and/or Step (iv) may be performed using any suitable vessel, such as a Petri dish or a cell culture flask, containing any growth medium as disclosed herein. In an embodiment, during step (ii) and/or step (iv), the *Bacillus Spp.* may be grown in a medium comprising yeast extract (YE) at a concentration lower than 2 mg/ml. In a preferred embodiment, the medium has YE at a concentration lower than 1.5 mg/ml, 1 mg/ml, 0.5 mg/ml, 0.25 mg/ml, 0.125 mg/ml, 0.1 mg/ml, 0.01 mg/ml, 0.001 mg/ml, 0.0001 mg/ml, or 0 mg/ml. In an embodiment, the medium in which the *Bacillus Spp.* is grown contains no YE.

In an embodiment, during step (ii) and/or step (iv), the *Bacillus Spp.* may be grown in a medium comprising thymine or thymidine. In a preferred embodiment, the thymine or thymidine may be present between 0.1 µg/ml and 1000 µg/ml, 1 µg/ml and 500 µg/ml, less than or equal to 300 µg/ml, 10 µg/ml and 200 µg/ml, 20 µg/ml and 100 µg/ml, 30 µg/ml and 80 µg/ml, 35 µg/ml and 65 µg/ml, 45 µg/ml and 55 µg/ml, or preferably approximately 50 µg/ml.

In an embodiment, during step (ii) and/or step (iv), the growth medium is any suitable growth medium, for instance Spizizen's Minimal Medium (SMM).

In an embodiment, the amount of trimethoprim present in step (iv) is higher than the amount of trimethoprim present in step (ii).

In an embodiment, during step (ii), the medium may comprise trimethoprim at a concentration of 0.001 µg/ml to 20 µg/ml, 0.01 µg/ml to 15 µg/ml, 0.1 µg/ml to 10 µg/ml, 1 µg/ml to 5 µg/ml, 2 µg/ml to 4 µg/ml, or preferably approximately 3 µg/ml. In a preferred embodiment, the concentration of trimethoprim is lower than the concentration of trimethoprim in step (iv).

In an embodiment, step (ii) may comprise or consist of 12 hours to 168 hours, 24 hours to 144 hours, 48 hours to 120 hours, 60 hours to 108 hours, or 72 hours to 96 hours of growth.

In an embodiment, cells carrying an insertion at the *thyA* locus may be verified by the ability to grow at 37_{°}C with or without thymine or thymidine but the inability to grow at 46_{°}C unless supplemented with thymine or thymidine. A further, or alternative, verification is to amplify, by PCR, the presence of the chimeric gene from transformants using primers annealing to the *thyA* sequences. In some embodiments, the colonies may be screened and separated into two types: i) large opaque colonies (2-3 mm after 72h-96h of growth), ii) translucent, smaller colonies (1 mm after 72h-96h of growth). In some embodiments, type (i) colonies may be selected.

In an embodiment, step (iii) may be performed by the transformation of *Bacillus Spp.* with a plasmid, which may be linearised, which may comprise at least a portion of the left arm of a *Bacillus Spp. thyB* gene, a heterologous gene, and at least a portion of the right arm of a *Bacillus Spp. thyB* gene. Step (iii) may be performed with any vector as disclosed herein. Transformation may be carried out using electroporation.

In an embodiment, the *Bacillus Spp.* may be prepared or made electro-competent using a method that involves culturing *Bacillus Spp.* in medium that contains yeast extract (YE) at a concentration lower than 2 mg/ml. In a preferred embodiment, the medium has YE at a concentration lower than 1.5 mg/ml, 1 mg/ml, 0.5 mg/ml, 0.25 mg/ml, 0.125 mg/ml, 0.1 mg/ml, 0.01 mg/ml, 0.001 mg/ml, 0.0001 mg/ml, or o mg/ml. In an embodiment, the medium in which the *Bacillus Spp.* is grown contains no YE. In a most preferred embodiment, the *Bacillus Spp.* is a strain carrying a *thyA* insertion as generated by steps (i) and (ii).

In an embodiment, YE is present at a concentration lower than 2 mg/ml, any concentration disclosed herein, or preferably not present in the media used at any stage of the process.

In an embodiment, step (iv) may be performed using medium which may comprise trimethoprim at a concentration of less than or equal to 80 µg/ml, less than or equal to 40 µg/ml, less than or equal to 30 µg/ml, 0.01 µg/ml to 30 µg/ml, 0.1 µg/ml to 20 µg/ml, 1 µg/ml to 15 µg/ml, 2 µg/ml to 10 µg/ml, 5 µg/ml to 7 µg/ml, or preferably approximately 6 µg/ml. In a preferred embodiment the concentration of trimethoprim is higher than the concentration of trimethoprim in step (ii).

Casamino acids (CAA) are produced by the acid hydrolysis of casein. The typical composition is as follows, although these numbers are approximate and variations in composition are acceptable for use with the invention: Ala 2.8%, Arg 3.6%, Asp 6.3%, Cystine 0.3%, Glu 21.1%, Gly 2.2%, His 2.7%, Ile 5.6%, Leu 8.4%, Lys 7.5%, Met 2.7%, Phe 4.6%, Pro 9.9%, Ser 5.6%, Thr 4.2%, Trp 1.1%, Tyr 6.1%, Val 5.0%. CAA may not contain tryptophan.

In an embodiment, during step (iv), the *Bacillus Spp.* may be grown in a medium comprising casamino acids (CAA). The medium may comprise CAA at 0.0001% to 5%, 0.001% to 2%, 0.01% to 1%, 0.05% to 0.5%, 0.1% to 0.3%, or preferably approximately 0.2% (w/v).

In an embodiment, during step (iv), the *Bacillus Spp.* may be grown in a medium supplemented with at least one amino acid. In other embodiments the medium may comprise any naturally occurring amino acid or amino acids, and optionally all naturally occurring amino acids or all naturally occurring amino acids except tryptophan. The medium may comprise serine, glycine, cysteine, methionine, any combination thereof, or all four amino acids. The medium may comprise any individual amino acid at a concentration equivalent to medium containing CAA at 0.0001% to 5%, 0.001% to 2%, 0.01% to 1%, 0.05% to 0.5%, 0.1% to 0.3%, or preferably approximately 0.2% (w/v). Thus, in a preferred embodiment, the medium may comprise serine, glycine, cysteine, and/or methionine at a concentration equivalent to medium containing CAA at approximately 0.2% (w/v). In another preferred embodiment, the medium may comprise the same amino acids as medium containing CAA at approximately 0.2% (w/v).

In an embodiment, step (iv) may comprise or consist of 6 hours to 144 hours, 12 hours to 120 hours, 24 hours to 96 hours, 36 hours to 72 hours, or approximately 48 hours of growth.

In an embodiment, cells carrying two insertions may be verified by the inability to grow at 37_{°}C or 46_{°}C unless supplemented with thymine or thymidine. A further or alternative verification is to amplify, by PCR, the presence of the chimeric genes from transformants using primers annealing to the *thyA* and *thyB* sequences.

Any heterologous gene may be used for the gene insertion into the or each thymidylate synthase gene. In particular embodiments, the heterologous genes are fused to genes encoding spore coat proteins, for instance CotA, CotB, CotC, CotD, CotE, CotF, or CotG *of B. subtilis.* The spore protein used may be chosen depending on the particular bacterium being modified, and the appropriate gene used. Appropriate spore coat proteins for use with *Clostridium Spp.* include CotA, CotB, CotC, CotD, CotE, CotF, CdeC, BclA1, BclA2, or BclA₃ (53, 54, 55).

In some embodiments, a heterologous gene may be inserted into the *thyA* gene and the same heterologous gene may be inserted into the *thyB* gene. In other embodiments, a different heterologous gene may be inserted into the *thyA* gene from that inserted into the *thyB* gene.

In some embodiments, the resultant spore produced by the process may carry a heterologous protein for use in vaccination. The resultant spore may carry a heterologous protein which is displayed as an active enzyme. The resultant spore may carry a heterologous protein allowing the conjugation of proteins to the spore surface.

After generation and selection of a strain comprising the heterologous genes that are unable to proliferate in the absence of thymine or thymidine, the process may comprise cultivating the strain. The cultivation may be on any media comprising thymine or thymidine, YE at a concentration less than 2 mg/ml or absent, and at least one amino acid or any mixture as disclosed herein. The concentrations may be any as disclosed herein.

Thus, according to a particular embodiment of the invention, there is provided a process for introducing at least one heterologous gene into *B. subtilis* and rendering the *B. subtilis* unable to proliferate in the absence of thymine or thymidine, preferably wherein the process does not involve the introduction of an antibiotic resistance gene, comprising:
(i) introducing a heterologous gene into the *thyA* gene or *thyB* gene within *B. subtilis;*
(ii) growing said *B. subtilis* in the presence of trimethoprim and either thymine or thymidine, and YE at a concentration lower than 2 mg/ml, optionally wherein no YE is present;
(iii) introducing a heterologous gene into the other of the *thyA* gene or *thyB* gene within said *B. subtilis;*
(iv) growing said *Bacillus Spp.* in the presence of trimethoprim and either thymine or thymidine, and YE at a concentration lower than 2 mg/ml, optionally wherein no YE is present.

In a preferred embodiment, YE is present at a concentration lower than 2 mg/ml, preferably o mg/ml, during all steps following step (i). In a preferred embodiment, thymine or thymidine is present for all cultures following step (i). In a preferred embodiment, a heterologous gene is introduced into the *thyA* gene during step (i) and a heterologous gene is introduced into the *thyB* gene during step (iii).

Thus, according to a particular embodiment of the invention, there is provided a process for introducing at least one heterologous gene into *B. subtilis* and rendering the *B. subtilis* unable to proliferate in the absence of thymine or thymidine, preferably wherein the process does not involve the introduction of an antibiotic resistance gene, comprising:
(i) introducing a heterologous gene into the *thyA* gene or the *thyB* gene within *B. subtilis;*
(ii) growing said *B. subtilis* in the presence of trimethoprim, and in the presence of either thymine or thymidine;
(iii) introducing a heterologous gene into the other of the *thyA* gene or *thyB* gene within said *B. subtilis;*
(iv) growing said *Bacillus Spp.* in the presence of trimethoprim and either thymine or thymidine, and at least one amino acid, optionally wherein CAA is present.

In a preferred embodiment, thymine or thymidine is present for all cultures following step (i). In a preferred embodiment, a heterologous gene is introduced into the *thyA* gene during step (i) and a heterologous gene is introduced into the *thyB* gene during step (iii).

According to a second example, there is provided a bacterial spore obtained or obtainable by the process of the first aspect.

Preferably, the bacterial spore is *Bacillus spp.* and comprises non-functional *thyA* and *thyB* genes, preferably due to the introduction of heterologous genes therein. In a third example, there is provided a bacterial spore comprising at least one heterologous gene and unable to proliferate in the absence of thymine or thymidine, wherein the bacterial spore is produced by a process comprising:
(i) introducing a heterologous gene into a thymidylate synthase gene within a spore-forming bacterium;
(ii) growing said spore-forming bacterium in the presence of trimethoprim; and
(iii) forming a bacterial spore from said spore-forming bacterium.

In a preferred embodiment, the bacterial spore does not comprise an antibiotic resistance gene.

In a preferred embodiment, the bacterial spore may be *Bacillus Spp.* The *Bacillus spp.* bacterium may be *B. subtilis, B. cereus, B. pumilus, B. amyloliquefaeceans, B. coagulans, B. clausii, B. licheniformis or B. megaterium.* Most preferably, the *Bacillus spp.* bacterium is *B. subtilis.* In another example, the bacterial spore may be *Clostridium Spp.* The *Clostridium spp.* bacterium may be C. *difficile, C. perfringens, C. tetani,* C. *botulinum, C. acetobutylicum, C. cellulolyticum,* C. *novyi* or C. *thermocellum.* Most preferably, the *Clostridium spp.* bacterium is *Clostridium difficile.*

In an embodiment, there is provided a bacterial spore comprising at least one heterologous gene and unable to proliferate in the absence of thymine or thymidine, wherein the bacterial spore is produced by any process as disclosed herein, or using any culture or conditions as disclosed.

According to a particular embodiment of the invention, there is provided a *Bacillus Spp.* spore comprising at least one heterologous gene and unable to proliferate in the absence of thymine or thymidine, wherein the *Bacillus Spp.* spore is produced by a process comprising:
(i) introducing a heterologous gene into the *thyA* gene or the *thyB* gene within a *Bacillus Spp.;*
(ii) growing said *Bacillus Spp.* in the presence of trimethoprim and in the presence of thymine or thymidine, optionally in the presence of YE at a concentration lower than 2 mg/ml, preferably wherein no YE is present;
(iii) introducing a heterologous gene into the other of the *thyA* gene or *thyB* gene within said *Bacillus Spp.;*
(iv) growing said *Bacillus Spp.* in the presence of trimethoprim, optionally at a higher concentration than in step (ii), and in the presence of thymine or thymidine,
   optionally in the presence of YE at a concentration lower than 2 mg/ml, preferably wherein no YE is present,
   optionally in the presence of at least one amino acid, optionally wherein CAA is present; and
(v) forming bacterial spores from said *Bacillus Spp.*

In a preferred embodiment, a heterologous gene is introduced into the *thyA* gene during step (i) and a heterologous gene is introduced into the *thyB* gene during step (iii).

According to a fourth example, there is provided a vector for use in introducing a heterologous gene into the *thyA* gene within a spore-forming bacterium.

In an embodiment, the vector may comprise at least a portion of the 5' portion of a *Bacillus Spp. thyA* gene, a heterologous gene, and at least a portion of the 3' portion of a *Bacillus Spp. thyA* gene.

In an embodiment, the vector may comprise the left and right arms of a *Bacillus Spp. thyA* gene. The arms may flank a multiple cloning site. For instance, the vector may include the left (e.g. 900 bp) and right (e.g. 950 bp) arms of the *B. subtilis thyA* gene.

The length of the flanking arms may be adjusted for the length of the heterologous sequence to be inserted. Longer flanking arms (e.g. ~1500bp) may be necessary for large inserts.

The vectors may comprise a heterologous gene inserted at the multiple cloning site. The heterologous gene may be a chimeric gene comprising a spore coat protein fused to a heterologous gene. For instance, the spore coat protein may be CotB or CotC of *B*. *subtilis.* The vectors may comprise: the left arm of a *Bacillus Spp. thyA* gene, a spore coat protein, a multiple cloning site allowing insertion of a heterologous gene such that it is fused to the spore coat protein, and the right arm of a *Bacillus Spp. thyA* gene.

In one purely illustrative embodiment, the left arm of *thyA* may be encoded by the nucleic acid sequence provided herein as SEQ ID NO: 3 as follows:

CAGATCATATAAGGAATGAACCGCTGCCAAATATCATAAAAAAGTTGTTAATGATCAAATGAATGAAATTAGAGAGAA
TTTATTTTAAAGAAAGCCCAATTGCACATGGACAAATGACAATTGATATTGAGGTAAAATACAATGCATTAAATCAGA
AAAAGCTATTGGAGGATTTAATGTGTTTAGACAATTTCCAATTTGGTATACACAAACACCTGACTATTTGAATTTTTA
TGTACCGCAATATCAAACCATTTCGTATAATCCTCAACAATGTTATCAACGGTGTATGTACCAAACTGGCGGTAACTA
TGAGCTATGTGACAGACTATGTTATGGAGAAATACAGGTGTAAAAGAGGGGGATTAACTCCTCTTTAAACACACAGTG
AGTGGAATAAGATCCTCACTTTATCTGCAAGTGCTTAGTATTTGCGATAATATTGCATTCGTAATAAATTATGCTTAG
CAACTGAAAATGAAAGAAGGATATGAATAGTCATGACGCAATTCGATAAACAATACAATTCAATTATAAAGGATATTA
TCAATAATGGAATCTCAGACGAAGAGTTTGATGTAAGAACCAAGTGGGACTCAGATGGAACACCGGCACATACTCTAA
GTGTAATCAGTAAGCAAATGAGATTCGACAACTCAGAGGTTCCGATTTTAACGACAAAAAAGGTTGCCTGGAAAACAG
CCATTAAAGAGTTGCTCTGGATTTGGCAGCTGAAATCTAATGATGTTAATGATTTAAACATGATGGGCGTCCATATTT
GGGATCAGTGGAAACAAGAAGACGGAACCATCGGACATGCATATGGATTTCAGCTGGGGAAGAAAAACAGAAGTCTAA
ATGGAGAAAAAGTGGATCAGGTAGACTATCTTCTTCATCAAT
[SEQ ID NO: 3]

The bold sequence represents the *thyA* coding sequence, the non-bold sequence represents the upstream region. Preferably, therefore, the vector comprises a nucleic acid sequence substantially as set out in SEQ ID NO: 3, or a fragment or variant thereof.

In one purely illustrative embodiment, the right arm of thyA may be encoded by the nucleic acid sequence provided herein as SEQ ID NO: 4 as follows:

TGAAGAACAATCCATCTTCACGCAGACACATTACAATGCTGTGGAATCCTGATGAATTAGACGCAATGGCCTTAACGC
CATGTGTATACGAGACACAATGGTACGTTAAACATGGGAAACTCCACCTTGAGGTAAGAGCACGGAGCAATGATATGG
CATTGGGAAATCCATTCAATGTATTCCAGTATAATGTGTTGCAGCGCATGATTGCTCAAGTGACTGGTTATGAGCTTG
GTGAATATATCTTTAACATTGGGGATTGCCATGTGTACACACGTCATATAGACAATTTGAAAATTCAAATGGAAAGAG
AACAGTTTGAAGCACCTGAACTATGGATCAATCCTGAAGTGAAAGATTTTTATGACTTTACCATTGATGATTTCAAGT
TAATCAACTATAAACATGGGGACAAGCTTTTATTTGAGGTAGCGGTTTAATGCTGCCTTTTTATTGTGCAGTGAATAG
ATAGCAGGTATCCTAATTTCATTAAGCAATCTGGAAGATGAATAAAAATTGAAGGACAAACACGTATAATACATAAAA
AAGATTAACTCTACAGTTAATCTTTTTTATTCAGAAGAAAATATCCTAACTTTGAAACTAAATACAAAGTAAAAGCAA
TCATTACAGTTCTAGATATTACAATTCCATGAATAGCTAGATCATATCCAGCAGGTATCAACGCATTTGTATTACACA

TAAAATATATAGATATTAGAAGTGCTACAATAACTAAAATCATTCCAAAAAGACTTGTTTTTTCATATTTCATACCAA
TTTCCACCCTTATTAAAGTTAGGTTTAAACAAAAGAGCTGAAGAAACGAACTATGACCAGTATGCTCCAAGGAAAACC
GCCAGACAATGCTGGCGGCTTTTTGCTGCTTCGTTTATTTATTAACAGAGATCGTAACGTTATTTCCTGCAACTGAAA
CCTTTGCGAAATCC
[SEQ ID NO: 4]

The bold sequence represents the *thyA* coding sequence, the non-bold sequence represents the downstream region. Preferably, therefore, the vector comprises a nucleic acid sequence substantially as set out in SEQ ID NO: 4 or a fragment or variant thereof.

Any suitable multiple cloning site could be used in a vector of the invention. For instance, in an embodiment, the multiple cloning site may be encoded by the nucleic acid sequence provided herein as SEQ ID NO: 5 as follows:
AGCTTGCATGCCTGCAGGTCGACTCTAGAGGATCCCCCGGGGGTACCGAGCTCGAATTC [SEQ ID NO: 5]

Preferably, therefore, the vector comprises a nucleic acid sequence substantially as set out in SEQ ID NO: 5 or a fragment or variant thereof.

In one purely illustrative embodiment, the *thyA* vector may comprise a nucleic acid sequence which may be encoded by the nucleic acid sequence provided herein as SEQ ID NO: 6 as follows:

CAGATCATATAAGGAATGAACCGCTGCCAAATATCATAAAAAAGTTGTTAATGATCAAATGAATGAAATTAGAGAGAA
TTTATTTTAAAGAAAGCCCAATTGCACATGGACAAATGACAATTGATATTGAGGTAAAATACAATGCATTAAATCAGA
AAAAGCTATTGGAGGATTTAATGTGTTTAGACAATTTCCAATTTGGTATACACAAACACCTGACTATTTGAATTTTTA
TGTACCGCAATATCAAACCATTTCGTATAATCCTCAACAATGTTATCAACGGTGTATGTACCAAACTGGCGGTAACTA
TGAGCTATGTGACAGACTATGTTATGGAGAAATACAGGTGTAAAAGAGGGGGATTAACTCCTCTTTAAACACACAGTG
AGTGGAATAAGATCCTCACTTTATCTGCAAGTGCTTAGTATTTGCGATAATATTGCATTCGTAATAAATTATGCTTAG
CAACTGAAAATGAAAGAAGGATATGAATAGTCATGACGCAATTCGATAAACAATACAATTCAATTATAAAGGATATTA
TCAATAATGGAATCTCAGACGAAGAGTTTGATGTAAGAACCAAGTGGGACTCAGATGGAACACCGGCACATACTCTAA
GTGTAATCAGTAAGCAAATGAGATTCGACAACTCAGAGGTTCCGATTTTAACGACAAAAAAGGTTGCCTGGAAAACAG
CCATTAAAGAGTTGCTCTGGATTTGGCAGCTGAAATCTAATGATGTTAATGATTTAAACATGATGGGCGTCCATATTT
GGGATCAGTGGAAACAAGAAGACGGAACCATCGGACATGCATATGGATTTCAGCTGGGGAAGAAAAACAGAAGTCTAA
ATGGAGAAAAAGTGGATCAGGTAGACTATCTTCTTCATCAATAAGCTTGCATGCCTGCAGGTCGACTCTAGAGGATCC
CCCGGGGGTACCGAGCTCGAATTCTGAAGAACAATCCATCTTCACGCAGACACATTACAATGCTGTGGAATCCTGATG
AATTAGACGCAATGGCCTTAACGCCATGTGTATACGAGACACAATGGTACGTTAAACATGGGAAACTCCACCTTGAGG
TAAGAGCACGGAGCAATGATATGGCATTGGGAAATCCATTCAATGTATTCCAGTATAATGTGTTGCAGCGCATGATTG
CTCAAGTGACTGGTTATGAGCTTGGTGAATATATCTTTAACATTGGGGATTGCCATGTGTACACACGTCATATAGACA
ATTTGAAAATTCAAATGGAAAGAGAACAGTTTGAAGCACCTGAACTATGGATCAATCCTGAAGTGAAAGATTTTTATG
ACTTTACCATTGATGATTTCAAGTTAATCAACTATAAACATGGGGACAAGCTTTTATTTGAGGTAGCGGTTTAATGCT
GCCTTTTTATTGTGCAGTGAATAGATAGCAGGTATCCTAATTTCATTAAGCAATCTGGAAGATGAATAAAAATTGAAG
GACAAACACGTATAATACATAAAAAAGATTAACTCTACAGTTAATCTTTTTTATTCAGAAGAAAATATCCTAACTTTG
AAACTAAATACAAAGTAAAAGCAATCATTACAGTTCTAGATATTACAATTCCATGAATAGCTAGATCATATCCAGCAG
GTATCAACGCATTTGTATTACACATAAAATATATAGATATTAGAAGTGCTACAATAACTAAAATCATTCCAAAAAGAC
TTGTTTTTTCATATTTCATACCAATTTCCACCCTTATTAAAGTTAGGTTTAAACAAAAGAGCTGAAGAAACGAACTAT
GACCAGTATGCTCCAAGGAAAACCGCCAGACAATGCTGGCGGCTTTTTGCTGCTTCGTTTATTTATTAACAGAGATCG
TAACGTTATTTCCTGCAACTGAAACCTTTGCGAAATCC
[SEQ ID NO: 6]

Preferably, therefore, the vector comprises a nucleic acid sequence substantially as set out in SEQ ID NO: 6, or a fragment or variant thereof. Example primer annealing sites, which could be used to verify insertion of the heterologous gene, are shown in bold and underlined.

According to a fifth example, there is provided a vector for use in introducing a heterologous gene into the *thyB* gene within a spore-forming bacterium.

In an embodiment, the vector may comprise at least a portion of the 5' portion of a *Bacillus Spp. thyB* gene, a heterologous gene, and at least a portion of the 3' portion of a *Bacillus Spp. thyB* gene.

In an embodiment, the vector may comprise the left and right arms of a *Bacillus Spp. thyB* gene. The arms may flank a multiple cloning site. For instance, the vector may include the left (900bp) and right (1.1 kb) arms of the *B. subtilis thyB* gene.

The length of the flanking arms may be adjusted for the length of the heterologous sequence to be inserted. Longer flanking arms (e.g. ~1500bp) may be necessary for large inserts.

The vectors may include a heterologous gene inserted at the multiple cloning site. The heterologous gene may be a chimeric gene comprising a spore coat protein fused to a heterologous gene. For instance, the spore coat protein may be CotB or CotC of B. *subtilis.* The vectors may comprise: the left arm of a *Bacillus Spp. thyB* gene, a spore coat protein, a multiple cloning site allowing insertion of a heterologous gene such that it is fused to the spore coat protein, and the right arm of a *Bacillus Spp. thyB* gene.

In a purely illustrative embodiment, the left arm of *thyB* may be encoded by the nucleic acid sequence provided herein as SEQ ID NO: 7, as follows:

CCAAATCTGCCGCTCAGTGTTTGCATGGAGAATGTAGAAAAAGTCCTGAACAAACGTGAAATTATTCATGCTGTTTTG
ACAGGCCTTGCACTCGATCAGCTTGCAGAACAGAAACTTCTCCCCGAACCGCTGCAGCACCTTGTTGAAACGGATGAA
CCGCTTTACGGCATAGATGAAATTATCCCGCTTTCAATCGTTAATGTGTACGGGTCGATCGGTTTGACCAATTTCGGT
TATTTGGATAAAGAGAAGATTGGAATTATTAAGGAACTTGATGAAAGTCCAGACGGTATTCACACCTTTTTGGATGAT
ATTGTGGCAGCTCTTGCTGCAGCAGCGGCGAGCAGAATTGCACATACGCATCAGGATCTGCAAGATGAAGAAAAAGAA
CAGGATGAAAAGCCTGTCGTCAGCTGACTATAAAAAAATCATTTCTGGGTTCAGAAATGATTTTTTATTGTGTTACAC
TACTAGAAGACTACTTTTAAAGGATGAAAAAAATGAAACAGTATAAGGATTTCTGCAGACATGTTTTAGAGCATGGTG
AGAAAAAGGGAGACCGGACTGGGACCGGAACAATCAGCACTTTCGGATATCAAATGAGATTTAATTTACGGGAAGGCT
TTCCGATGCTCACCACTAAAAAACTCCACTTTAAATCAATTGCGCATGAACTGCTGTGGTTCTTAAAAGGAGATACGA
ATGTACGCTATCTGCAGGAAAACGGAGTGCGAATCTGGAATGAGTGGGCTGATGAAAACGGTGAACTTGGACCTGTAT

ATGGCTCCCAATGGCGTTCTTGGCGGGGAGCTGATGGAGAAACCATTGATCAAATTTCCCGTCTTATTGAAGATATTA
AAACAAATCCGAACTCCAGACGCTTAATCGTCAGCGCCTGGA
[SEQ ID NO: 7]

The bold sequence represents the *thyB* coding sequence, the non-bold sequence represents the upstream region. Preferably, therefore, the vector comprises a nucleic acid sequence substantially as set out in SEQ ID NO: 7, or a fragment or variant thereof.

In one purely illustrative embodiment, the right arm of *thyB* may be encoded by the nucleic acid sequence provided herein as SEQ ID NO: 8, as follows:

ATGTTGGTGAAATTGATAAAATGGCGTTGCCGCCGTGCCATTGCCTGTTCCAATTCTATGTGTCTGACGGCAAGCTGT
CCTGTCAGCTGTATCAGCGCTCTGCCGATGTTTTCTTAGGTGTGCCGTTTAATATTGCATCTTATGCCCTCCTAACCA
TGATCATTGCTCATGTGACTGGGCTTGAACCGGGCGAGTTCATCCATACGTTTGGTGATGTTCATATTTACCAAAATC
ATATTGAACAAGTCAATTTGCAGCTGGAAAGAGATGTTAGACCGCTTCCGCAGCTTCGTTTCGCCAGAAAGGTTGATT
CTATTTTTAACTTTGCATTTGAGGACTTTATCATCGAGGATTATGATCCGCATCCTCATATAAAAGGGGCGGTCAGCG
TATGATTTCATTCATTTTTGCGATGGATGCCAACAGGCTTATCGGCAAAGACAATGATTTGCCGTGGCATTTGCCCAA
TGATCTTGCATACTTTAAGAAAATAACATCGGGCCATTCAATCATTATGGGCCGGAAAACATTTGAATCGATCGGACG
TCCGCTTCCAAATCGGAAAAATATTGTCGTTACCTCAGCGCCGGATTCAGAATTTCAGGGATGCACGGTTGTCAGTTC
ATTAAAGGATGTACTGGACATTTGTTCAGGCCCTGAAGAATGCTTTGTGATCGGAGGGGCTCAGCTCTATACGGACCT
GTTCCCTTATGCGGACAGACTGTATATGACGAAAATTCATCACGAGTTTGAGGGTGACCGTCACTTTCCTGAATTTGA
TGAATCCAATTGGAAGCTGGTTTCTTCTGAGCAGGGGACCAAAGACGAAAAAAACCCGTATGATTACGAATTTCTAAT
GTATGAAAAAAAGAAATCTTCTAAAGCGGGAGGATTTTAATTGGTTCGCTACAGCCTTCTAGTGGTTTATATTGTGTA
TATGCTGTTAAAAAATATGAAACAATTATTTAATCAAACAATGCTCGATCCCCGTCTGTCATACAAAAAACAGATGGC
TCTTGTGTACGAACAGCCAAAGGCGTTTTTAGAAGGCTGTATCGGCATCTCCGGTTCAGTTGTGACGATCCATCAGCC
AGA
[SEQ ID NO: 8]

The bold sequence represents the *thyB* coding sequence, the non-bold sequence represents the downstream region. Preferably, therefore, the vector comprises a nucleic acid sequence substantially as set out in SEQ ID NO: 8, or a fragment or variant thereof.

The above illustrative embodiments of both *thyA* and *thyB* sequences are intended as an example. As the skilled person would be aware, the sequences will vary between species, in particular there is a great deal of variability between the upstream and downstream sequences.

Any suitable multiple cloning site could be used in a vector of the invention. For instance, in an embodiment, the multiple cloning site may be encoded by the nucleic acid sequence provided herein as SEQ ID NO: 9, as follows: AAGCTTGCATGCCTGCAGGTCGACTCTAGAGGATCCCCCGGGGGTACCGAGCTCGAATTCTG [SEQ ID NO: 9]

Preferably, therefore, the vector comprises a nucleic acid sequence substantially as set out in SEQ ID NO: 9, or a fragment or variant thereof.

In one embodiment, the *thyB* vector may comprise a nucleic acid sequence which may be encoded by the nucleic acid sequence provided herein as SEQ ID NO: 10, as follows:

CCAAATCTGCCGCTCAGTGTTTGCATGGAGAATGTAGAAAAAGTCCTGAACAAACGTGAAATTATTCATGCTGTTTTG
ACAGGCCTTGCACTCGATCAGCTTGCAGAACAGAAACTTCTCCCCGAACCGCTGCAGCACCTTGTTGAAACGGATGAA
CCGCTTTACGGCATAGATGAAATTATCCCGCTTTCAATCGTTAATGTGTACGGGTCGATCGGTTTGACCAATTTCGGT
TATTTGGATAAAGAGAAGATTGGAATTATTAAGGAACTTGATGAAAGTCCAGACGGTATTCACACCTTTTTGGATGAT
ATTGTGGCAGCTCTTGCTGCAGCAGCGGCGAGCAGAATTGCACATACGCATCAGGATCTGCAAGATGAAGAAAAAGAA
CAGGATGAAAAGCCTGTCGTCAGCTGACTATAAAAAAATCATTTCTGGGTTCAGAAATGATTTTTTATTGTGTTACAC
TACTAGAAGACTACTTTTAAAGGATGAAAAAAATGAAACAGTATAAGGATTTCTGCAGACATGTTTTAGAGCATGGTG
AGAAAAAGGGAGACCGGACTGGGACCGGAACAATCAGCACTTTCGGATATCAAATGAGATTTAATTTACGGGAAGGCT
TTCCGATGCTCACCACTAAAAAACTCCACTTTAAATCAATTGCGCATGAACTGCTGTGGTTCTTAAAAGGAGATACGA
ATGTACGCTATCTGCAGGAAAACGGAGTGCGAATCTGGAATGAGTGGGCTGATGAAAACGGTGAACTTGGACCTGTAT
ATGGCTCCCAATGGCGTTCTTGGCGGGGAGCTGATGGAGAAACCATTGATCAAATTTCCCGTCTTATTGAAGATATTA
AAACAAATCCGAACTCCAGACGCTTAATCGTCAGCGCCTGGAAAGCTTGCATGCCTGCAGGTCGACTCTAGAGGATCC
CCCGGGGGTACCGAGCTCGAATTCTGATGTTGGTGAAATTGATAAAATGGCGTTGCCGCCGTGCCATTGCCTGTTCCA
ATTCTATGTGTCTGACGGCAAGCTGTCCTGTCAGCTGTATCAGCGCTCTGCCGATGTTTTCTTAGGTGTGCCGTTTAA
TATTGCATCTTATGCCCTCCTAACCATGATCATTGCTCATGTGACTGGGCTTGAACCGGGCGAGTTCATCCATACGTT
TGGTGATGTTCATATTTACCAAAATCATATTGAACAAGTCAATTTGCAGCTGGAAAGAGATGTTAGACCGCTTCCGCA
GCTTCGTTTCGCCAGAAAGGTTGATTCTATTTTTAACTTTGCATTTGAGGACTTTATCATCGAGGATTATGATCCGCA
TCCTCATATAAAAGGGGCGGTCAGCGTATGATTTCATTCATTTTTGCGATGGATGCCAACAGGCTTATCGGCAAAGAC
AATGATTTGCCGTGGCATTTGCCCAATGATCTTGCATACTTTAAGAAAATAACATCGGGCCATTCAATCATTATGGGC
CGGAAAACATTTGAATCGATCGGACGTCCGCTTCCAAATCGGAAAAATATTGTCGTTACCTCAGCGCCGGATTCAGAA
TTTCAGGGATGCACGGTTGTCAGTTCATTAAAGGATGTACTGGACATTTGTTCAGGCCCTGAAGAATGCTTTGTGATC
GGAGGGGCTCAGCTCTATACGGACCTGTTCCCTTATGCGGACAGACTGTATATGACGAAAATTCATCACGAGTTTGAG
GGTGACCGTCACTTTCCTGAATTTGATGAATCCAATTGGAAGCTGGTTTCTTCTGAGCAGGGGACCAAAGACGAAAAA
AACCCGTATGATTACGAATTTCTAATGTATGAAAAAAAGAAATCTTCTAAAGCGGGAGGATTTTAATTGGTTCGCTAC
AGCCTTCTAGTGGTTTATATTGTGTATATGCTGTTAAAAAATATGAAACAATTATTTAATCAAACAATGCTCGATCCC
CGTCTGTCATACAAAAAACAGATGGCTCTTGTGTACGAACAGCCAAAGGCGTTTTTAGAAGGCTGTATCGGCATCTCC
GGTTCAGTTGTGACGATCCATCAGCCAGA
[SEQ ID NO: 10]

Preferably, therefore, the vector comprises a nucleic acid sequence substantially as set out in SEQ ID NO: 10, or a fragment or variant thereof. Example primer annealing sites, which could be used to verify insertion of the heterologous gene, are shown in bold and underlined.

According to a sixth example, there is provided a kit comprising reagents for use with the processes of the invention.

In an embodiment, the kit may comprise any medium as disclosed herein, for instance medium comprising YE at a concentration lower than 2 mg/ml, or comprising no YE. The kit may comprise trimethoprim. The kit may comprise medium comprising trimethoprim, optionally two mediums one with a higher level of trimethoprim than the other. The kit may comprise CAA. The kit may comprise one or more amino acids, or any combination or concentration as disclosed herein. The kit may comprise instructions describing the use of the kit or describing any process as disclosed herein.

The kit may comprise at least one container for performing a process as disclosed herein, for instance a petri dish.

In an embodiment, the kit may comprise one or more vectors of the invention. The kit may comprise a vector for use in introducing a heterologous gene into the *thyA* gene within a spore-forming bacterium and/or a vector for use in introducing a heterologous gene into the *thyB* gene within a spore-forming bacterium. The kit may comprise a nucleic acid sequence including a heterologous gene, such as a gene for a vaccine, for a conjugation protein, or an enzyme. The kit may comprise a nucleic acid sequence including a spore coat protein. The spore coat protein may be configured to be easily attachable to a heterologous gene, for instance by proximity to a multiple cloning site.

It will be appreciated that the invention extends to any nucleic acid or peptide or variant, derivative or analogue thereof, which comprises substantially the amino acid or nucleic acid sequences of any of the sequences referred to herein, including functional variants or functional fragments thereof. The terms "substantially the amino acid/nucleotide/peptide sequence", "functional variant" and "functional fragment", can be a sequence that has at least 40% sequence identity with the amino acid/nucleotide/peptide sequences of any one of the sequences referred to herein, for example 40% identity with the sequence identified as SEQ ID Nos: 1 - 19.

Amino acid/polynucleotide/polypeptide sequences with a sequence identity which is greater than 50%, more preferably greater than 65%, 70%, 75%, and still more preferably greater than 80% sequence identity to any of the sequences referred to are also envisaged. Preferably, the amino acid/polynucleotide/polypeptide sequence has at least 85% identity with any of the sequences referred to, more preferably at least 90%, 92%, 95%, 97%, 98%, and most preferably at least 99% identity with any of the sequences referred to herein.

The skilled technician will appreciate how to calculate the percentage identity between two amino acid/polynucleotide/polypeptide sequences. In order to calculate the percentage identity between two amino acid/polynucleotide/polypeptide sequences, an alignment of the two sequences must first be prepared, followed by calculation of the sequence identity value. The percentage identity for two sequences may take different values depending on:- (i) the method used to align the sequences, for example, ClustalW, BLAST, FASTA, Smith-Waterman (implemented in different programs), or structural alignment from 3D comparison; and (ii) the parameters used by the alignment method, for example, local vs global alignment, the pair-score matrix used (e.g. BLOSUM62, PAM250, Gonnet etc.), and gap-penalty, e.g., functional form and constants.

Having made the alignment, there are many different ways of calculating percentage identity between the two sequences. For example, one may divide the number of identities by: (i) the length of shortest sequence; (ii) the length of alignment; (iii) the mean length of sequence; (iv) the number of non-gap positions; or (iv) the number of equivalenced positions excluding overhangs. Furthermore, it will be appreciated that percentage identity is also strongly length dependent. Therefore, the shorter a pair of sequences is, the higher the sequence identity one may expect to occur by chance.

Hence, it will be appreciated that the accurate alignment of protein or DNA sequences is a complex process. The popular multiple alignment program ClustalW (Thompson et al., 1994, Nucleic Acids Research, 22,4673-4680; Thompson et al., 1997, Nucleic Acids Research, 24, 4876-4882) is a preferred way for generating multiple alignments of proteins or DNA in accordance with the invention. Suitable parameters for ClustalW may be as follows: For DNA alignments: Gap Open Penalty = 15.0, Gap Extension Penalty = 6.66, and Matrix = Identity. For protein alignments: Gap Open Penalty = 10.0, Gap Extension Penalty = 0.2, and Matrix = Gonnet. For DNA and Protein alignments: ENDGAP = -1, and GAPDIST = 4. Those skilled in the art will be aware that it may be necessary to vary these and other parameters for optimal sequence alignment.

Preferably, calculation of percentage identities between two amino acid/polynucleotide/polypeptide sequences may then be calculated from such an alignment as (N/T)*100, where N is the number of positions at which the sequences share an identical residue, and T is the total number of positions compared including gaps and either including or excluding overhangs. Preferably, overhangs are included in the calculation. Hence, a most preferred method for calculating percentage identity between two sequences comprises (i) preparing a sequence alignment using the ClustalW program using a suitable set of parameters, for example, as set out above; and (ii) inserting the values of N and T into the following formula:- Sequence Identity = (N/T)^{∗}100.

Alternative methods for identifying similar sequences will be known to those skilled in the art. For example, a substantially similar nucleotide sequence will be encoded by a sequence which hybridises to the sequences shown or their complements, under stringent conditions. By stringent conditions, the inventors mean the nucleotide hybridises to filter-bound DNA or RNA in 3x sodium chloride/sodium citrate (SSC) at approximately 45°C followed by at least one wash in 0.2x SSC/0.1% SDS at approximately 20-65°C. Alternatively, a substantially similar polypeptide may differ by at least 1, but less than 5, 10, 20, 50 or 100 amino acids from the sequences shown.

Due to the degeneracy of the genetic code, it is clear that any nucleic acid sequence described herein could be varied or changed without substantially affecting the sequence of the protein encoded thereby, to provide a functional variant thereof. Suitable nucleotide variants are those having a sequence altered by the substitution of different codons that encode the same amino acid within the sequence, thus producing a silent change. Other suitable variants are those having homologous nucleotide sequences but comprising all, or portions of, sequence, which are altered by the substitution of different codons that encode an amino acid with a side chain of similar biophysical properties to the amino acid it substitutes, to produce a conservative change. For example, small non-polar, hydrophobic amino acids include glycine, alanine, leucine, isoleucine, valine, proline, and methionine. Large non-polar, hydrophobic amino acids include phenylalanine, tryptophan and tyrosine. The polar neutral amino acids include serine, threonine, cysteine, asparagine and glutamine. The positively charged (basic) amino acids include lysine, arginine and histidine. The negatively charged (acidic) amino acids include aspartic acid and glutamic acid. It will therefore be appreciated which amino acids may be replaced with an amino acid having similar biophysical properties, and the skilled technician will know the nucleotide sequences encoding these amino acids.

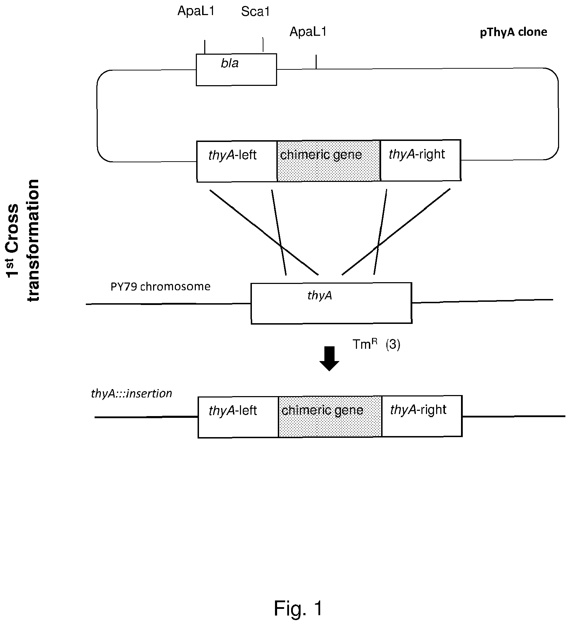

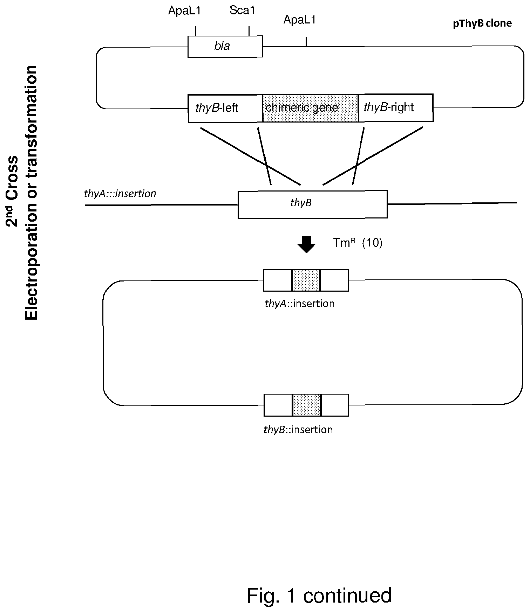

For a better understanding of the invention, and to show how embodiments of the same may be carried into effect, reference will now be made, by way of example, to the accompanying diagrammatic drawings, in which:-
**Figure 1** is a graphical representation of the strain constructions. To construct ectopic insertions at the *thyA* and *thyB* loci of *B*. *subtilis* two steps are required. In step 1 a pThyA plasmid carrying a chimeric gene is linearised (ApaII digestion) and introduced into cells of a wild type *B. subtilis* strain (in this case strain PY₇₉) by DNA-mediated transformation. Tm^{R} transformants are selected on SMM agar containing trimethoprim (3 µg/ml) and thymine (50 µg/ml) and carry an insertion of homologous *thyA* DNA together with the chimeric gene, by marker replacement, as shown. In the second step linearised plasmid DNA of a pThyB vector carrying the same or a different chimeric gene is introduced into cells of the *thyA* insertion strain created in the 1^{st} step. Selection for Tm^{R} is made on SMM + CAA agar containing trimethoprim (> 6 µg/ml) and thymine (50 µg/ml);

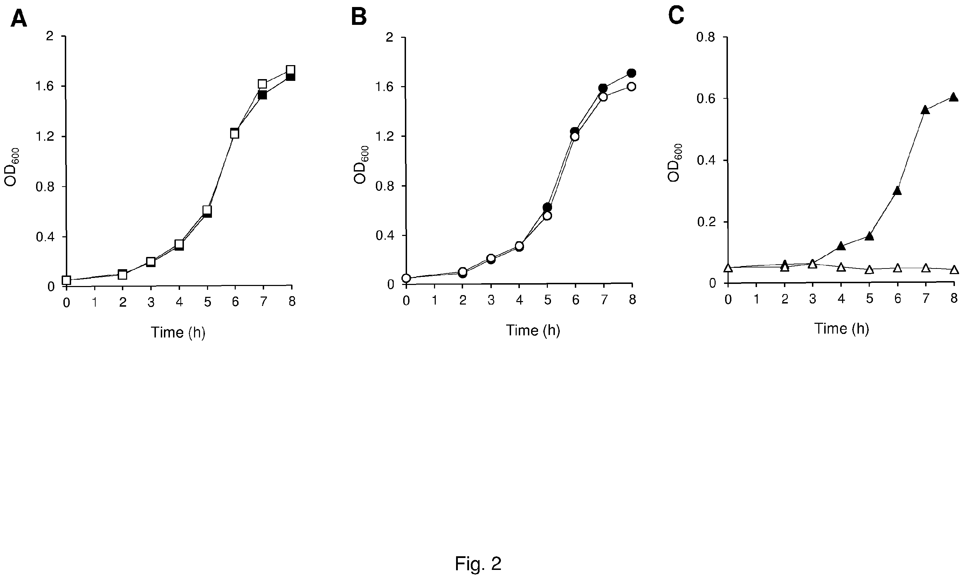

**Figure 2** is a graphical representation of growth of strains in minimal media. Wild type PY₇₉ *(panel A),* SH₁₃ *thyA: :cotB-tcdA₂₆₋₃₉ (panel B)* and SH₁₄ *thyA::cotB-tcdA₂₆₋39 thyB: :cotC-tcdA₂₆₋₃₉ (panel* C) were grown in SMM medium at 37_{°}C with (filled symbols) or without (open symbols) thymine supplementation (50 µg/ml);

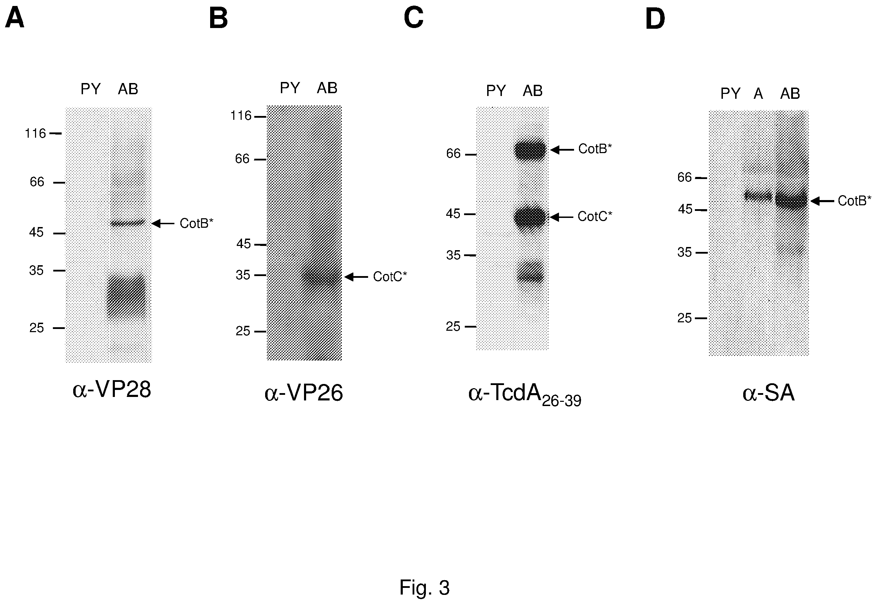

**Figure 3** shows spore coat expression. *B. subtilis* strains carrying insertions at the *thyA* and *thyB* loci were examined by Western blotting of spore coat proteins extracted from preparations of pure spores. Each panel shows bands obtained in extracts of wild type spores (PY₇₉) or spores carrying *thyA* and *thyB* insertions (AB) or, in addition in *panel D,* spores carrying only a *thyA* insertion (A). *Panels A* and *B* show analysis of SH₁₂ *thyA::cotB-vp26 thyB: :cotC-vp28* with anti-VP28 *(panel A*) and anti-VP26 *(panel B)* antibodies. *Panel* C shows analysis of SH₁₄ *thyA: :cotB-tcdA₂₆₋₃₉ thyB::cotC-tcdA₂₆₋₃₉. Panel D,* SH16 *thyA::cotB-SA thyB::cotB-SA.* Bands corresponding to the chimeric proteins are indicated (*) with the relevant coat protein anchor (CotB or CotC). The protein loaded per well corresponded to an extraction from 2 X 10⁸ spores (see Methods);

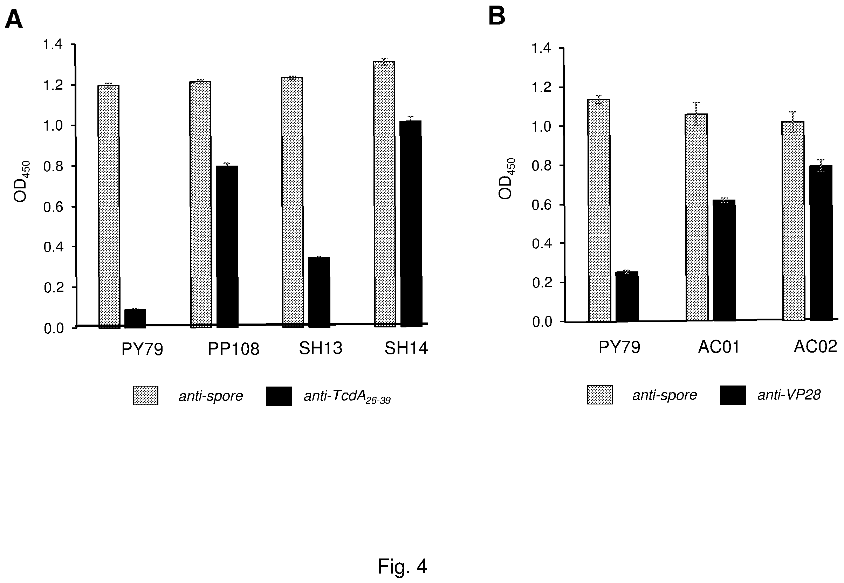

**Figure 4** is a graphical representation of surface expression determined by "Whole Spore ELISA". *Panel A;* Microtiter plates were coated with spores (2 X 10⁸/well) of PY₇₉ (spo⁺), PP108 *(amyE::cotC-tcdA_{26~39} thrC::cotB-tcdA₂₆₋₃₉),* SH₁₃ *(thyA::cotB-tcdA₂₆₋₃₉)* and SH₁₄ *(thyA: :cotB-tcdA₂₆₋₃₉ thyB: :cotCt-tcdA₂₆₋₃₉)* and then probed with either anti-spore (1:1,000) or anti-TcdA₂₆₋₃₉ (1:500) rabbit PAbs. Secondary PAbs were 1:5,000 and naive serum was used for comparison, and basal levels were subtracted. *Panel B;* as for *panel A* but using spores of PY₇₉, ACo1 *(thyA: :cotB-vp28)* and ACo2 *(thyA: :cotB-vp28 thyB: :cotB-vp28)* probed with either anti-spore (1:1,000) or anti-VP28 (1:300) rabbit PAbs;

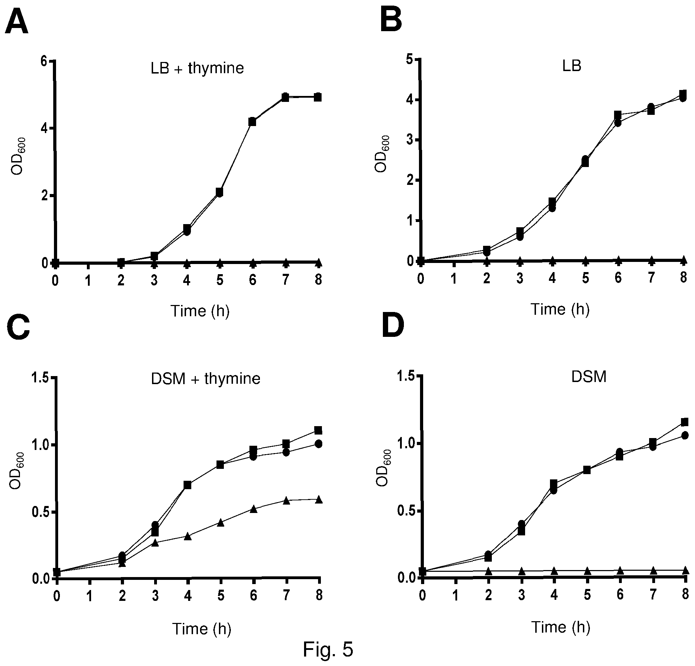

**Figure 5** is a graphical representation of growth of strains in rich media. Growth at 37_{°}C of PY₇₉ (■), SH₁₃ (•) and SH₁₄ (▲) were made in: *panel A,* LB + thymine (50 µg/ml); panel B, LB; *panel C,* DSM + thymine (50 µg/ml) and *panel D,* DSM;

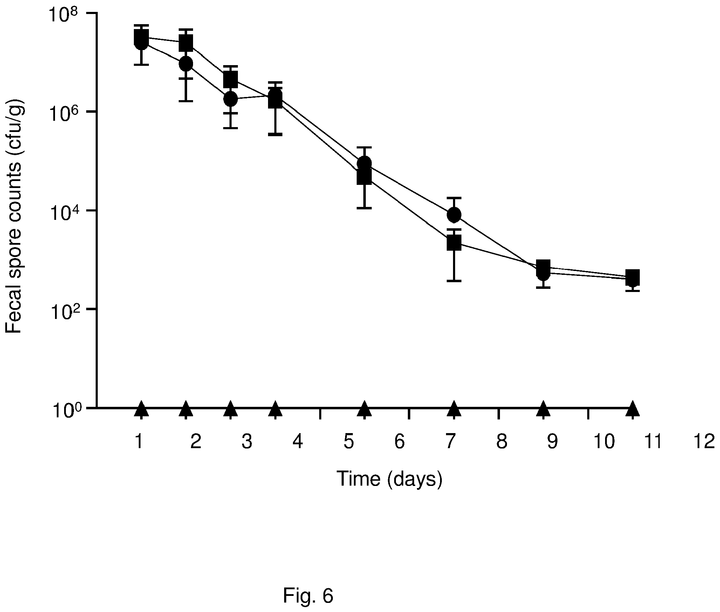

**Figure 6** is a graphical representation of survival in the murine GI-tract. Mice (n= 5_{/}gp) were given a single oral dose of spores (2 X 10⁹) of SH₁₄ carrying insertions in the *thyA* and *thyB* loci or SH250, an essentially wild type strain tagged with a chloramphenicol resistance marker. The numbers of heat resistant spores in faeces was determined for 12 days post-dosing. SH₁₄ numbers were determined on agar supplemented with trimethoprim and thymine (■). In the absence of thymine no CFU was detectable (◆). SH250 CFU was determined on agar supplemented with chloramphenicol (●);

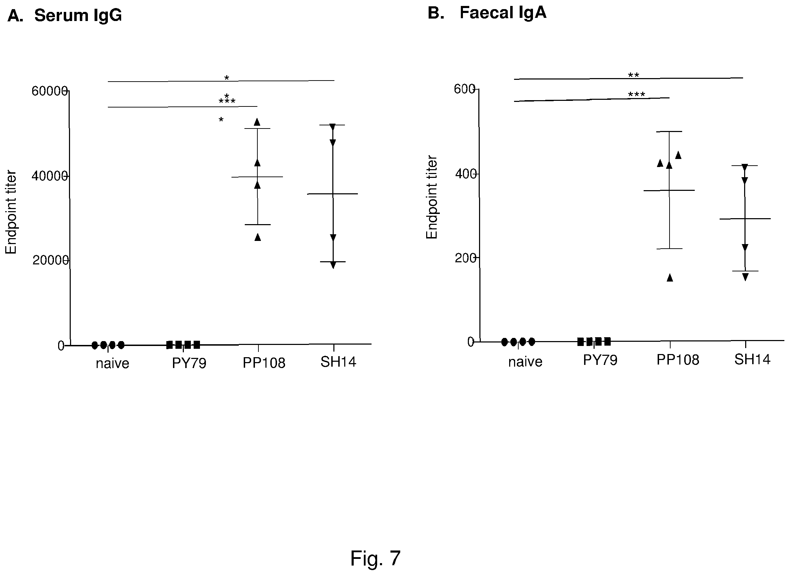

**Figure 7** is a graphical representation showing the immunogenicity of SH₁₄ spores expressing the C. *difficile* TcdA₂₆₋₃₉ antigen. Spores (5 X 10¹⁰) of SH₁₄ *(thyA::cotB-tcdA₂₆₋₃₉ thyB::cotC-tcdA₂₆₋₃₉),* PP108 *(amyE: :cotC-tcdA₂₆₋₃₉ thrC::cotB-tcdA₂₆₋₃₉*) and PY₇₉ (*spo*⁺) were administered to mice (n=4) by oral gavage on days 1, 14, 35 and 57. Serum IgG (*panel A*) and fecal IgA (*panel B*) specific to TcdA₂₆₋₃₉) was determined by ELISA and endpoint titers are shown. **, p < 0.005; *** p < 0.0002;

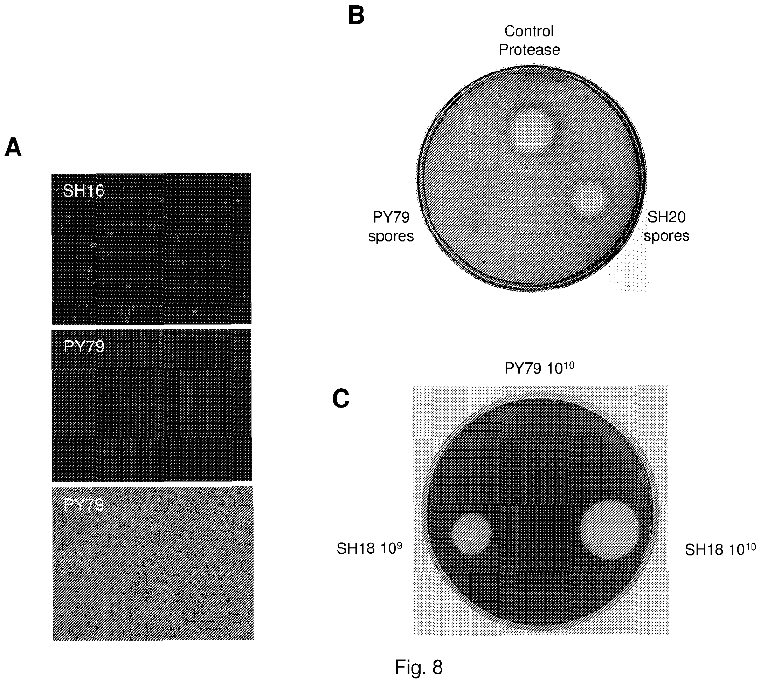

**Figure 8** is demonstrative of the conjugation of antibodies to spore-displayed streptavidin and enzyme display. **Panel A** shows visualisation of conjugation of anti-TcdA₂₆₋₃₉ antibodies to SH16 spores using immunofluorescence. As a control PY₇₉ spores lacking SA failed to conjugate. The phase contrast image confirms the presence of PY₇₉ spores. **Panel B** shows activity of subtilisin E displayed on the spore surface.
Casein agar was used to visualise protease activity. Plates were spotted with 20µl of suspensions of SH20 *(thyA::aprE thyB::MCS)* or PY₇₉ spores. In each case the inoculum carried 5 X 10⁸ spores. As a positive control 0.02 units of *Streptomyces griseus* protease (Sigma P5147) was applied. After 24h incubation at 37_{°}C plates were stained with Bromocresol green and incubated 30 min. at RT to reveal zones of degradation. **Panel C** shows starch plates stained with Lugol solution. Suspensions of pure spores of SH18 (10⁹ and 10¹⁰ cfu) or PY₇₉ (10¹⁰ cfu) were spotted on plates and
incubated for 48h. Plates carried three antibiotics to prevent bacterial growth. Spores displaying active amylase produced a zone of clearing;

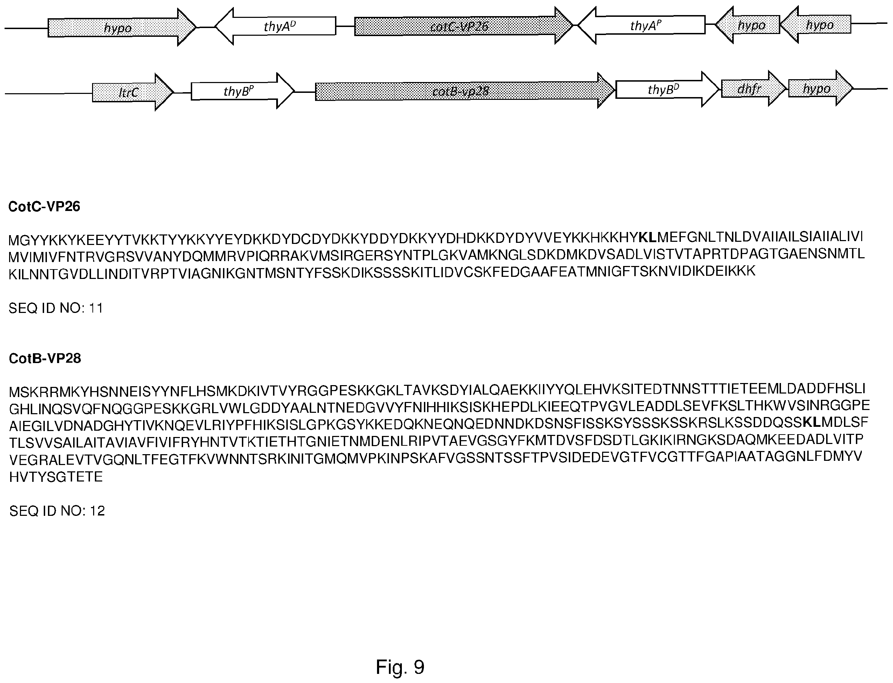

**Figure 9** shows schematic representations of thy insertions in SH₁₂ (*thyA::cotC-vp26 thyB::cotB-vp28*)*.* The proximal (^{P}) and distal (^{D}) segments of *thyA* and *thyB* flanking the chimeric gene insertion are shown. ORFS shown are: *hypo,* hypothetical coding ORFs, *dhfr,* dihydrofolate reductase, *ItrC* (Low temperature requirement C protein);

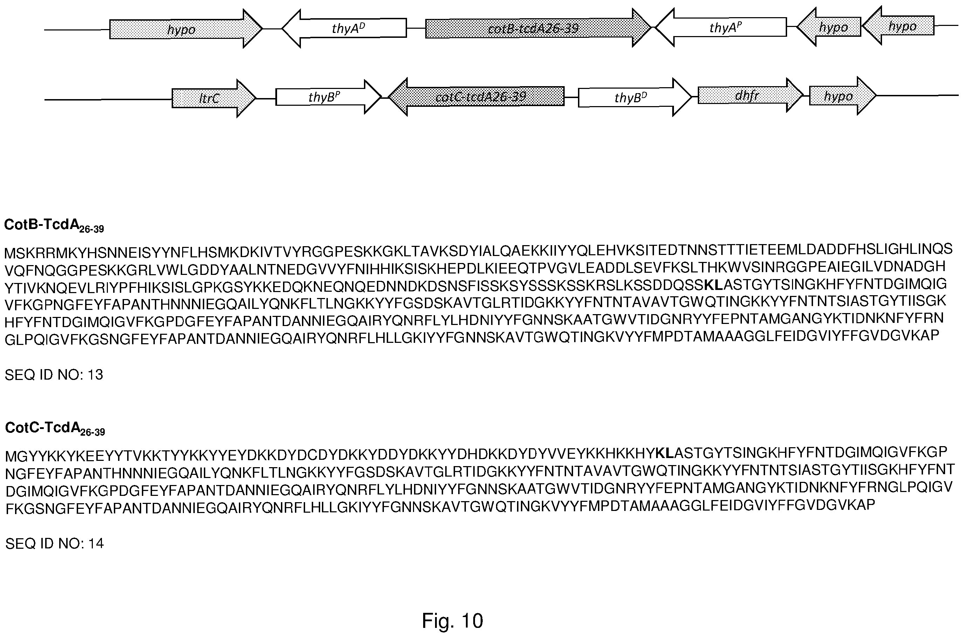

**Figure 10** shows schematic representations of thy insertions in SH₁₄ *(thyA: :cotB-tcdA₂₆₋₃₉ thyB: :cotC-tcdA₂₆₋₃₉)* . The proximal (^{P}) and distal (^{D}) segments of *thyA* and *thyB* flanking the chimeric gene insertion are shown. ORFS shown are: *hypo,* hypothetical coding ORFs, *dhfr,* dihydrofolate reductase, *ltrC* (low temperature requirement C protein);

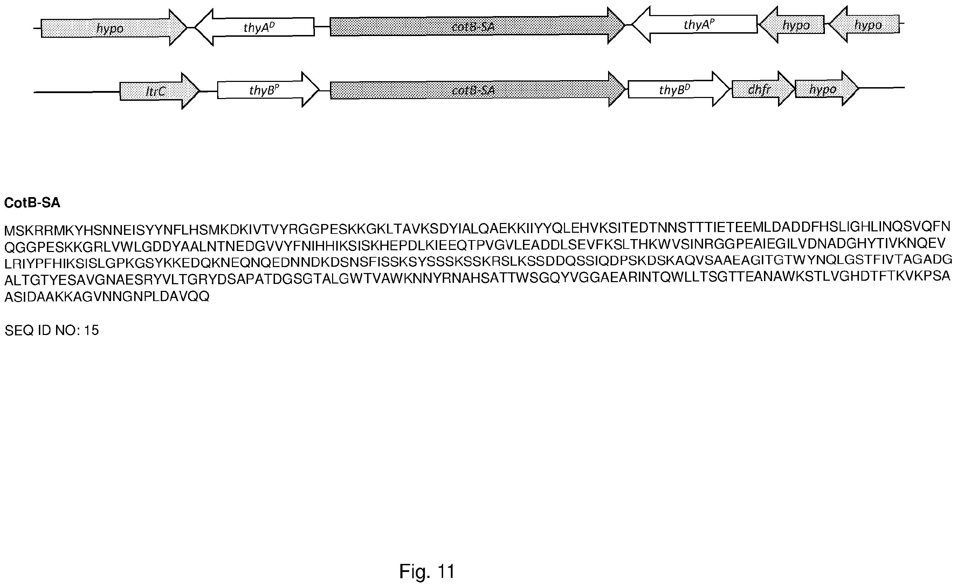

**Figure 11** shows schematic representations of thy insertions in SH16 *(thyA::cotB-SA thyB::cotB-SA)* . The proximal (^{P}) and distal (^{D}) segments of *thyA* and *thyB* flanking the chimeric gene insertion are shown. ORFS shown are: *hypo,* hypothetical coding ORFs, *dhfr,* dihydrofolate reductase, *ltrC* (Low temperature requirement C protein);

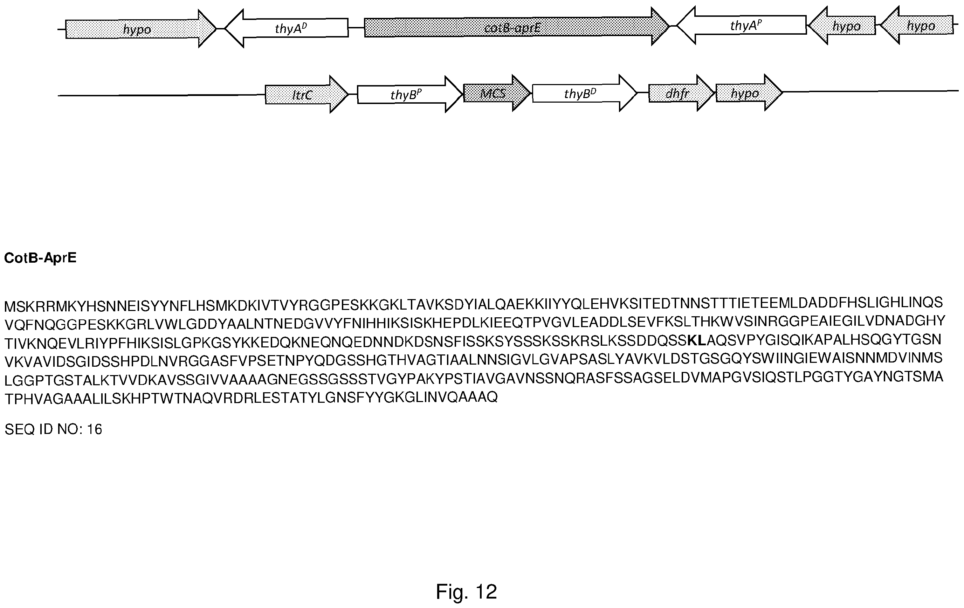

**Figure 12** shows schematic representations of thy insertions in SH₂₀ *(thyA::cotB-aprE thyB::MCS).* The proximal (^{P}) and distal (^{D}) segments of *thyA* and *thyB* flanking the chimeric gene insertion are shown. ORFS shown are: *hypo,* hypothetical coding ORFs, *dhfr,* dihydrofolate reductase, *ltrC* (Low temperature requirement C protein);

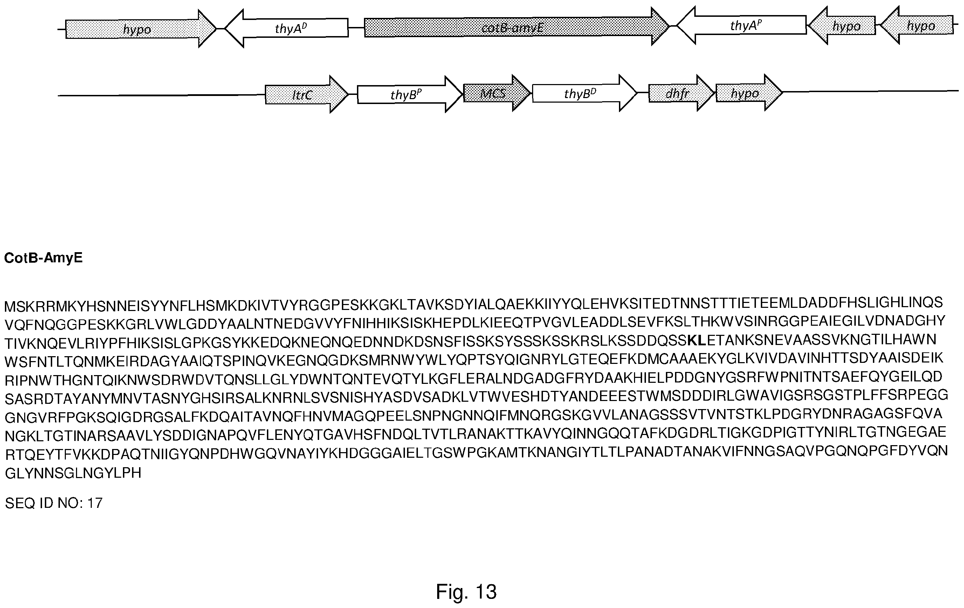

**Figure 13** shows schematic representations of thy insertions in SH18 *(thyA: :cotB-amyE* thyB::MCS).The proximal (^{P}) and distal (^{D}) segments of *thyA* and *thyB* flanking the chimeric gene insertion are shown. ORFS shown are: *hypo,* hypothetical coding ORFs, *dhfr,* dihydrofolate reductase, *ltrC* (Low temperature requirement C protein);

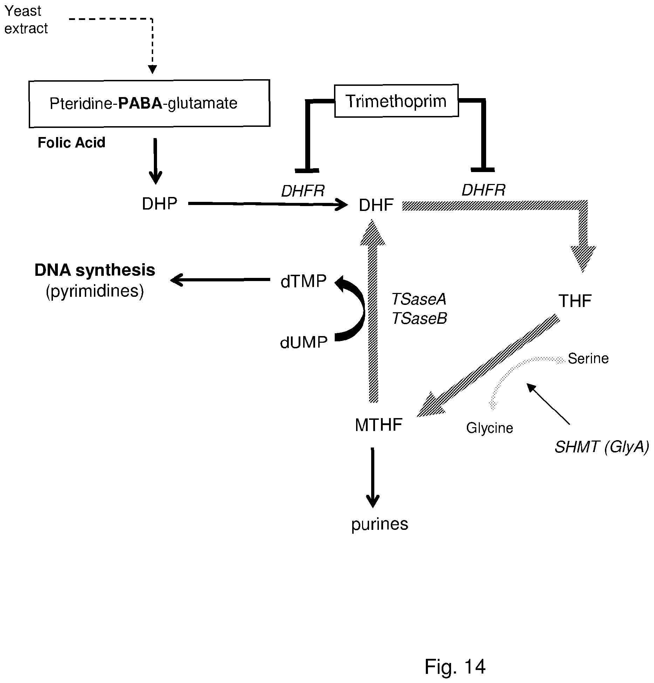

**Figure 14** is a schematic representation of the action of Trimethoprim on the Folate Pathway. Trimethoprim inhibits the enzyme dihydrofolic acid (DHFR) which is required for production of dihyrofolate (DHF) from dihydropterate (DHP) as well as for synthesis of tetrahydrofolic acid (THF). This prevents synthesis of methyleneterahydrofolic acid (MTHF) and synthesis of both purines and pyrimidines. In *B. subtilis,* two thymidylate synthetases (TS), TSase A and TSase B use MTHF to produce the pyrimidines thymidine and thymine. Inactivation of both TS enzymes is lethal unless exogenous thymine (or thymidine) is supplied. Also shown is *para-*aminobenzoic acid (PABA) which is a component of folic acid and SHMT (serine hydroxymethyltransferase);

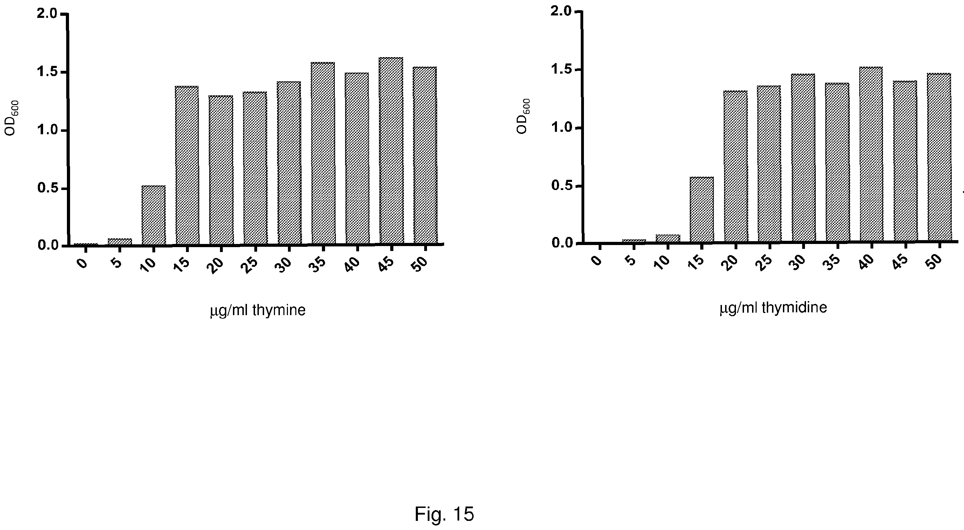

**Figure 15** shows a schematic representations of growth of SH₁₄ *(thyA::cotB-tcdA₂₆-₃₉; thyB::cotC-tcdA₂₆₋₃₉₎* in medium containing thymine or thymidine. SH₁₄ was grown in SMM containing 0.2% CAA with different concentrations of thymine or thymidine. Cultures incubated 18h at 37_{°}C after which ODs determined;

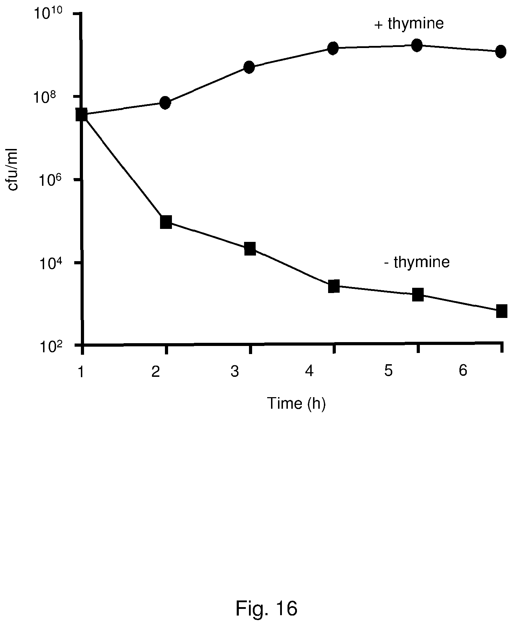

**Figure 16** shows schematic representations of loss of viability after removal of thymine. SH₁₄ *(thyA::cotB-tcdA₂₆₋₃₉ thyB::cotC-tcdA₂₆₋₃₉*) was grown (37_{°}C) in SOC medium containing trimethoprim (6 µg/ml) and thymine (50 µg/ml) overnight and then subcultured into fresh SOC supplemented with thymine (50 µg/ml). At an OD₆₀₀ of 0.5-0.6 the cells were harvested by centrifugation, washed 3X with PBS and suspended in 20ml of warm SOC medium. 10ml of this cell suspension was added to each of two flasks to which one thymine was added to give a final concentration of 50 µg/ml. The cultures were incubated at 37_{°}C with shaking in a water bath and viable cfu/ml determined at intervals by serial dilution and plating;

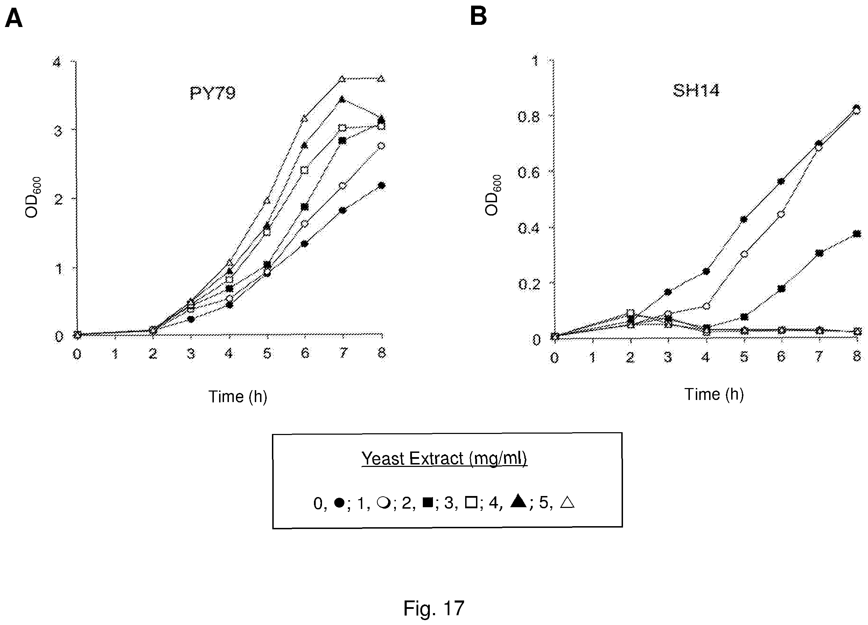

**Figure 17** shows schematic representations of growth of strains in rich media. Growth at 37_{°}C of PY₇₉ *(panel A),* and SH₁₄ *(thyA: : cotB-tcdA₂₆₋₃₉ thyB: :cotC-tcdA₂₆₋₃₉) (panel B*) were made in LB + thymine with different concentrations of yeast extract (mg/ml);

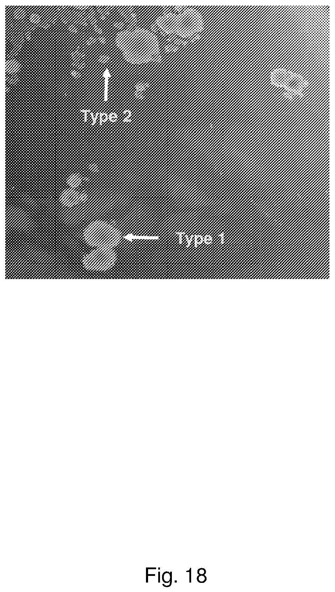

**Figure 18** shows colony types following the ₁^{st} Genetic Cross. After transformation two type of colonies are seen: large-opaque (Type 1) and small-translucent (Type 2). Only the opaque colonies could be used for electroporation for construction of the double insertion strain; and

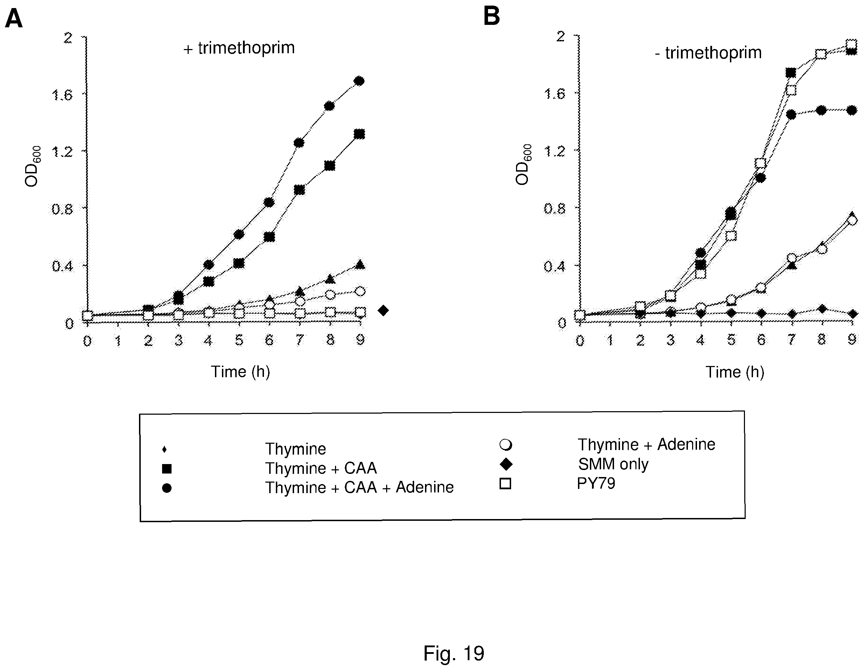

**Figure 19** shows schematic representations of growth of *thyA thyB* insertion strain in SMM media. Growth at 37_{°}C of SH₁₄ *(thyA: : cotB-tcdA₂₆₋₃₉ thyB::cotC-tcdA₂₆₋₃₉₎* in SMM medium using different supplements as indicated (thymine 50µg/ml, CAA (0.2% w/v,) Adenine (20µg/ml). Media was supplemented with trimethoprim (3µg/ml) in *panel A* and no trimethoprim in *panel B.* As a control *B. subtilis* strain PY₇₉ was also evaluated for growth in SMM with no supplements.

### Examples

Genetic manipulation of bacterial spores of the genus *Bacillus* has shown potential for vaccination and for delivery of drugs or enzymes. Remarkably, proteins displayed on the spore surface retain activity and generally are not degraded. The heat stability of spores coupled with their desiccation resistance make them suitable for delivery to humans or to animals by the oral route. Despite these attributes one regulatory obstacle has remained regarding the fate of recombinant spores shed into the environment as viable spores. The inventors have addressed the biological containment of spore GMOs by utilizing the concept of a 'thymine-less death', a phenomenon first reported six decades ago but which has never been successfully put into practice for introducing heterologous genes into spore-forming bacteria. Using *B. subtilis* the inventors have inserted chimeric genes in the two thymidylate synthase genes, *thyA* and *thyB* using a two-step process. Insertion first at *thyA* followed by *thyB* where insertion generates resistance to trimethoprim enabling selection of recombinants. Importantly, this method requires introduction of no new antibiotic resistance genes. Recombinant bacteria or vegetative cells have a strict dependence on thymine or thymidine and in their absence cells lyse and die. Insertions are stable and no evidence for suppression or reversion was evident. Using this system the inventors have successfully created a number of spore vaccines as well as spores displaying active enzymes.

### Materials and Methods

### Strains, media and general methods

PY₇₉ is a prototrophic strain of *B*. *subtilis* derived from the type strain 168 (28). PP108 *(amyE::cotC-tCdA₂₆₋₃₉ thrC::cotB-tcdA₂₆₋₃₉)* has been described elsewhere (4). SH250 is a prototrophic derivative of PY₇₉ carrying the *cat* gene (encoding resistance to chloramphenicol) inserted at the *amyE* locus. General methods for work with *B. subtilis* including the 'two-step transformation procedure' were performed as described previously (29). DSM (Difco Sporulation Medium) is a standard medium for growth and sporulation of *B*. *subtilis* (30). For western blotting purified spores were prepared and 2 X 10⁸ suspended in 40 µl Bolt LDS buffer (Life Tech.) and incubated at 95°C for 10 min. The spore suspension was centrifuged (10 min. 18,000g) and 20µl of supernatant run on a 12% SDS-PAGE gel.

### pThy vectors

pThyA (4,274 bp) carried a 1,910 bp segment comprising the left (900 bp) and right (950 bp) arms of the *B. subtilis thyA* gene surrounding a multiple cloning site (MCS) cloned into pMA-RQ (2,556 bp; Genscript, USA). Both arms carried additional proximal and distal DNA sequences adjacent to thyA. Similarly, pThyB1 (4,973 bp) carried a 2,057 bp segment comprising the left (900bp) and right (1.1 kb) arms of the B. *subtilis thyB* gene surrounding a MCS cloned into pBluescript SK(+) (2,958 bp). Plasmids carried the *bla* gene and the nucleotide sequences of the *thyA* and *thyB* segments are given in SEQ ID NO: 6 and SEQ ID NO: 10 (respectively) and shown schematically in **Figure 1**. pThyA and pThyB plasmids were constructed which carried chimeric genes inserted at the MCS sites of each vector. Chimeric genes (not optimised for codon usage) containing an in-frame fusion between the 5' segment of *B*. *subtilis cotB* or *cotC* to the *vp26, vp28, tcdA₂₆₋₃₉* or *SA* coding ORFs were first synthesised with suitable 5' and 3' ends for sub-cloning in the MCS of the pThyA and pThyB vectors. For two genes, *aprE* and *amyE* the inventors amplified from chromosomal DNA templates and then fused to *cotB.* The *aprE* gene (encoding subtilisin E) was PCR amplified from a *B. subtilis* strain (SG115) in our collection and the coding segment amplified lacked the N-terminal regions involved in protein secretion (pre) and activation (pro) (31). The *amyE* gene (encoding alpha amylase) was amplified from a lab strain of *Bacillus amyloliquefaciens* and was cloned devoid of the secretory signal sequence. The gene fusions used for this work are shown in **Figs. 9** to **13**.

*Construction* of *B*. *subtilis strains carrying thyA and thyB insertions* The procedure developed here consisted of two steps. In the first stage cells of a wild-type recipient strain (in the work described here the prototrophic strain PY₇₉ was used) were made competent using a 'two-step transformation' procedure described by Dubnau and Davidoff-Abelson (33) and in common use in *Bacillus* labs (33, 34). pThyA plasmids carrying the chimeric gene were linearised with either ApaLI or Seal digestion and cells plated on SMM (Spizizen's Minimal Medium (SMM) (29) agar supplemented with thymine (50 µg/ml) and trimethoprim (3 µg/ml). After 72h-96h of growth single colonies were colony purified and assessed for growth at 37_{°}C and 46_{°}C on SMM agar ± thymine (50 µg/ml) + trimethoprim (3 µg/ml). Cells carrying an insertion at the *thyA* locus would grow at 37_{°}C with or without thymine but were unable to grow at 46_{°}C unless supplemented with thymine. A further verification was to amplify, by PCR, the presence of the chimeric gene from transformants using primers annealing to the *thyA* sequences. Typically transformation frequencies were about 2 X 10³/µg of competent cells with about 15-20% of colonies carrying the correct insertion (see later for explanation). In the second stage, a linearised (ApaL1 or Sca1) pThyB plasmid carrying a chimeric gene was introduced into cells of the *thyA* insertion strain by electroporation. Electroporated cells were plated on SMM+CAA (SMM containing 0.2% (w/v) casamino acids (CAA)) containing thymine (50 µg/ml) and trimethoprim (6 µg/ml) and incubated at 37_{°}C for 48h. To confirm the presence of both a *thyA* and *thyB* insertion colonies were streaked on SMM+CAA agar ± thymine (50 µg/ml) and grown at 37°C. Cells carrying two insertions were unable to grow at both 37_{°}C and 46_{°}C unless supplemented with thymine. A final verification was made using PCR primers that amplified the two insertions. Using electroporation, integration frequencies were about 1 X 10³/µg of linear DNA with ~20% of trimethoprim resistance colonies carrying two insertions (*thyA* and *thyB).*

### Electroporation

The procedure used here was modified from established methods (35) for electroporation in *Bacillus* primarily with the use of SOC₂ medium that contained no yeast extract. SOC2 was tryptone (2% w/v), NaCl (10mM), KCl (2.5 mM), MgCl₂ (5 mM), MgSO₄.₇H₂O (5 mM) and glucose (20 mM). An overnight culture of the strain carrying a *thyA* insertion was sub-cultured in 25ml of SOC2 medium (supplemented with 0.5M sorbitol) to give a starting OD₆₀₀ of 0.2. The culture was grown at 37_{°}C to an OD₆₀₀ of 1.4, cooled on ice for 10 min. and then harvested by centrifugation at 4°C (5,000 x g, 5 min.). Cells were washed 4-times in ice-cold electroporation solution (0.5M sorbitol, 0.5M mannitol, 10% (v/v) glycerol) and suspended in 1.6ml of the same ice-cold solution. The cells were now electro-competent and ready for immediate use. Cells were kept on ice and used within 30 min. although aliquots could be stored at - 80°C. 1µl (~50ng) of linearised plasmid DNA was added to 60µl of electro-competent cells and the mixture transferred to a pre-chilled cuvette (1mm gap width) and incubated for 1.5 min. on ice. The cuvette was then placed inside the electroporator (BioRad GenePulser Xcell) and the following parameters used for electroporation: Voltage 2, 100V, resistance 200W, time, 5 milliseconds and number of pulses, 1. After electroporation 1ml of recovery medium (SOC₂ medium containing 0.5M sorbitol and 0.38M mannitol) was added to the cuvette and the mixture transferred to a 2ml Eppendorf and incubated for 3h in 37_{°}C after which cells were serially diluted and plated on appropriate selective media.

### Whole-spore ELISA

An ELISA method was used to detect surface exposed proteins as described previously (36). Microplate wells were coated with 50µl of a suspension of pure spores (2 X 10⁸ spores/well) which left overnight at 4°C. Plates were blocked with 2% (w/v) BSA for 1h at 37°C. Rabbit polyclonal antibodies recognizing either the heterologous antigen expressed on the spore surface or the whole *B. subtilis* spore were used as primaries with incubation for 2h at RT. Anti-rabbit IgG-horseradish peroxidase (HRP) conjugate (1:5,000 in PBS plus 0.05% Tween-20) was used as a secondary with 1h incubation at RT. TMB (3, 3', 5, 5'-tetramethylbenzidine) was used as substrate.

### Antibodies

Polyclonal antibodies to VP28 and TcdA₂₆₋₃₉ were raised in rabbits using four subcutaneous injections (1mg/dose, every 14 days). Recombinant proteins were complexed with Freund's adjuvant and serum purified using protein A chromatography. VP26 polyclonals were raised in mice using purified rVP26 protein (4 intra-peritoneal doses at 14-day intervals, 10µg/dose, with Freund's adjuvant).

### Animal studies

For immunogenicity studies mice (C₅₇ Black 6, female, age 7 weeks) were dosed with preparations of pure spores of PY₇₉ or SH₁₄. Immune responses to TcdA₂₆₋₃₉ in serum and faecal samples were made as described previously (4). For longevity studies Balb/c mice (female, age 7-8 weeks) were used. Mice (n=5) were administered a single dose of pure spores (2 X 10⁹) of SH₁₄ or SH250 by oral gavage. At times thereafter freshly voided faeces was collected (3-4 pellets), homogenised and for SH₁₄ faeces serial dilutions plated on (i) DSM and (iii) DSM + trimethoprim (6 µg/ml) + thymine (50 µg/ml) agar plates. SH250 faecal dilutions were plated on DSM plates containing chloramphenicol (5 µg/ml). Individual SH₁₄ colonies were randomly checked for the presence of the thy insertions using PCR.

### Conjugation to streptavidin

Polyclonal antibodies (rabbit; 100 µg) raised to rTcdA₂₆₋₃₉ protein were biotinylated using the Lightning-Link Rapid biotin conjugation kit type A (Innova Biosciences). Purified spores (1 X 10⁹) of strain PY₇₉ or spores expressing CotB-SA (SH16) in 200µl of PBS were mixed with 1µg of biotinylated antibody and incubated overnight at 4°C. Spores were then washed four-times with PBS and suspended in 1ml of PBS. ~3 X 10⁸ of conjugated spores were used to coat microplate wells which were then probed with an anti-rabbit IgG-horseradish conjugate (1:5,000 in PBS plus 0.05% Tween-20) with 1h incubation at RT followed by three washes before TMB (3,3',5,5'-tetramethylbenzidine) colour development. As a control PY₇₉ spores were taken through the same procedure. For immunoflourescence 5 X 10⁶ of treated spores of SH16 or PY₇₉ were added to microscope slides and allowed to air dry. After three washes with PBS slides were blocked with PBS containing 2% (w/v) BSA plus 0.05% (v/v) Tween-20 for 45 min. at 37°C. Biotinylated anti-TcdA₂₆₋₃₉ antibodies (1:300 diln.; 200µl) were added to slides, incubated for 30 min. at RT and then washed 3-times with PBS + 0.05% (v/v) Tween-20. Rabbit FITC serum (Sigma Fo382 at 1:200 diln.) was added and slides incubated for 30 min. at RT. Image analysis was done with an EVOS fl LED microscope.

### Toxin subtraction assays

*C. difficile* strain RT176 *(tcdA+ tcdB+)* was grown in TY broth (3% (w/v) tryptose, 2% (w/v) yeast extract and 0.1% (w/v) sodium thiodlycolate) for 24h at 37°C. The cell free supernatant was filter-sterilised and kept at 4°C till assay. The minimum lethal concentration (MLC) of supernatant required to cause 100% toxicity to HT29 cells was determined using 2-fold dilution and addition of the diluted lysate to HT29 cells followed by assessment of toxicity using a cell rounding assay (4). For the assay 10⁹ pure spores of SH16 spores conjugated to TcdA₂₆₋₃₉ polyclonal antibodies (see above) were added to 200 µl of 2% McCoy's medium containing the MLC of toxins (typically a 1/4000 dilution). The mixture was incubated for 5 min. at RT and then cytotoxicity assessed using HT29 cells and incubation for 24h. As a control PY₇₉ spores that had been mixed with TcdA₂₆₋₃₉ antibodies (see above) were used in parallel.

### Enzyme activity

Protease activity was determined using the Universal Protease Assay described by Sigma Aldrich on their web site using casein as a substrate. Casein agar was 1% (w/v) casein, 1% (w/v) skimmed milk, 1% (w/v) and 1.2% (w/v) agar technical No. 2, Oxoid). Amylase activity in liquid was measured as described (37). Production of active amylase was tested by applying suspensions of spores (volume of 20 µl) to agar plates carrying only soluble starch (1% w/v) and beef extract (0.3% w/v) and three antibiotics (trimethoprim 10 µg/ml, chloramphenicol 30 µg/ml and erythromycin 30 µg/ml). Antibiotics were used to prevent any bacterial growth on the plates ensuring activity arose from dormant spores only. Plates were incubated 48h at 37_{°}C after which the plate was flooded with Lugol solution (Sigma) for 2 min. to reveal zones of starch degradation.

### Example 1

### Rationale

An absolute requirement for thymine in *B. subtilis* requires two thymidylate synthases (TSase) encoded by the unlinked *thyA* (TSaseA) and *thyB* (TSaseB) genes (27). These genes are linked to the folate pathway and provide pyrimidines for cell growth **(****Fig. 14**). TSaseB is thermo-sensitive and retains only ~5-8% activity at a restrictive temperature of 46_{°}C. Thus, inactivation of the *thyA* locus requires supplementation with thymine (or thymidine) for growth at 46_{°}C. TSaseA is not thermo-sensitive and so inactivation of *thyB* allows cells to grow at an elevated temperature. Inactivation of both *thyA* and *thyB* however, produces an absolute requirement on thymine for growth at both 37_{°}C and 46_{°}C. As shown by Neuhard et al (27) inactivation of the thy genes produces resistance to the anti-folate drug trimethoprim (or aminopterin) since the need for dihydrofolate reductase (DHFR), the target for trimethoprim, is dispensed with. However, the level of resistance differs, with insertion at *thyA* producing a lower level of resistance than that found in a *thyA thyB* mutant (27). This attribute of differential resistance to trimethoprim enables a novel, two-step, ectopic cloning system to be designed **(****Fig. 1**). In the first step, a gene is introduced at the *thyA* locus followed, in the second step, by insertion at *thyB* using resistance to increasing concentrations of trimethoprim for positive selection.

To demonstrate proof of concept for ectopic cloning at the thy loci the inventors chose a number of heterologous genes whose products had previously been expressed on the spore surface. In each case expression had been achieved by fusion to a *B. subtilis* gene encoding a surface expressed spore coat protein (either CotB or CotC). The proteins chosen were VP26 and VP28, both envelope proteins of the shrimp virus WSSV (White Spot Syndrome Virus). When displayed on *B. subtilis* spores and incorporated in feed these recombinant spores have been shown to confer protection to shrimps challenged with WSSV (7, 8, 9). (ii) TcdA₂₆₋₃₉ encoding a C-terminal domain of *Clostridium difficile* toxin A (TcdA) expressed on the spore surface has been shown to confer protection to *C. difficile* infection (CDI) in hamsters (4, 38) dosed orally with these recombinant spores. (iii) Streptavidin (SA) when expressed on spores can be conjugated to the monoclonal antibody Cetuximab enabling targeting to colon cancer cells (15). (iv) Finally, two enzymes, subtilisin E (AprE), an alkaline protease and alpha amylase (AmyE) which are both enzymes of industrial importance and commonly incorporated in animal feed (32, 39).

### A two-step method for ectopic gene insertion

Two plasmids, pThyA and pThyB, were designed and synthesised that carry the complete *thyA* or *thyB* genes interrupted midpoint with a multiple cloning site (MCS) for insertion of heterologous DNA (Fig. **1**, SEQ ID NO: 6, and SEQ ID NO: 10). To ensure sufficient DNA homology to enable a double crossover recombination event, the left and right arms each carried the relevant thy segments as well as flanking upstream or downstream DNA (a total of ~900 bp proximal and distal). In-frame fusions of the *cotB* and *cotC* genes (encoding the spore coat anchors required for display of heterologous proteins) to *vp26, vp28, tcdA₂₆₋₃₉, SA, aprE* or *amyE* ORFs **(****Figs. 9 to 13**) were then cloned into the appropriate pThyA or pThyB vectors. The pThyA plasmids were next linearised and used to transform competent cells of the wild type strain PY₇₉ with selection for trimethoprim resistance on plates supplemented with trimethoprim (3 µg/ml) and thymine at 37°C. Transformants carrying *thyA* insertions could be recognised by their failure to grow at 46_{°}C without thymine. In the second step, for introduction of insertions at the *thyB* locus classical DNA-mediated transformation of competent cells with the pThyB plasmid proved inefficient with low integration frequencies. Instead, the inventors developed an electroporation method that reliably and reproducibly enabled introduction of pThyB plasmids at the *thyB* locus in *thyA*- strains (see Methods) using selection with a higher concentration of trimethoprim (6 µg/ml). Strains carrying insertions at *thyA* and *thyB* were verified by their failure to grow at both 37_{°}C and 46_{°}C in the absence of thymine. Using this two-step process the inventors successfully constructed a number of strains carrying insertions (*vp26, vp28, tcdA₂₆₋₃₉* and *SA)* at the *thyA* and *thyB* loci **(Table 1**). In addition, the inventors also made strains carrying single insertions of the *aprE* and *amyE* genes at the *thyA* locus. To create an absolute thymine dependence the inventors simply linearised an 'empty' pThyB plasmid and introduced this DNA into the *thyA* mutant selecting for trimethoprim at 6 µg/ml. Strains the inventors successfully constructed included monovalent (VP28, TcdA₂₆₋₃₉, streptavidin, subtilisin E and amylase) as well as divalent (expression of VP26 and VP28 on one spore) expression by fusion to one or two different spore coat anchors (CotB and CotC). For each strain constructed in **Table 1** the inventors confirmed by nucleotide sequence analysis the integrity of the *thyA* or *thyB* insertion.

The inventors examined growth of the insertions in minimal SMM medium at 37_{°}C and **Fig. 2** shows examples of a strain (SH₁₄) carrying *cotB-tcdA₂₆₋₃₉* and *cotC-tcdA₂₆₋₃₉* insertions at the *thyA* and *thyB* loci. As expected the *thyA* insertion strain grew normally with or without thymine **(****Fig. 2B**) and was indistinguishable from wild type PY₇₉ **(****Fig. 2A**). By contrast the *thyA thyB* insertion strain was thymine dependent but in the presence of thymine had reduced fitness as shown from the lower maximal OD **(****Fig. 2C**).

In addition, using whole genome sequencing the inventors confirmed that strain SH₁₂ *(thyA: :cotC-vp26 thyB::cotB-vp28)* and SH₁₄ *(thyA: :cotB-tcdA₂₆₋₃₉ thyA: :cotC-tcdA*_{*26-*39}) carried the specific insertions at the thy loci without any chromosomal abberations (not shown). Expression of chimeric proteins was confirmed by Western blotting of proteins extracted from purified spores **(****Fig. 3** shows blots for detection of the VP26, VP28, TcdA₂₆₋₃₉ and SA chimeras in SH₁₂, SH₁₄ and SH16) demonstrating bands of the correct mwt. A second method was ELISA detection of whole spores using polyclonal antibodies to the corresponding heterologous antigen and **Fig 4** shows surface detection of the TcdA₂₆₋₃₉ **(****Fig. 4A**) and VP28 **(****Fig. 4B**) antigens by whole-spore ELISA. As expected, levels of the TcdA₂₆₋₃₉ protein carried at both the *thyA* and *thyB* loci (SH₁₄ in **Fig. 4A**) was greater than expression at one locus (i.e., *thyA*, SH₁₃). In parallel the inventors also measured the abundance of TcdA₂₆₋₃₉ in spores of PP108 that carried the same antigen fused to CotB and CotC but inserted at the *thrC* and *amyE* loci respectively (4). Expression levels were somewhat lower but it should be noted that using an anti-spore PAb to measure levels of spore coat proteins expression levels were correspondingly reduced.

Both thymine and thymidine could be used for growth of strains carrying insertions in the *thyA* and *thyB* loci. Using SH₁₄ the inventors showed that for optimal growth 15 µg/ml of thymine or 20 µg/ml of thymidine was sufficient (Fig. **15**). This was significantly less than the 50 µg/ml reported before (27) and most probably reflects differences in strain backgrounds used. Cell carrying insertions at the *thyA* and *thyB* loci although able to grow in media supplemented with thymine underwent a massive loss in cell viability (~5 logs in 5h) when thymine was removed, a hallmark of a 'thymine-less death' (Fig. 16).

**Table 1: Phenotypes of B. subtilis recombinant strains**

| **Strain** | **Genotype** | **37°C¹** | | **46°C¹** | | **Tm MIC²** | **Sporulation³** | | |
|---|---|---|---|---|---|---|---|---|---|
| | | **- thy** | **+ thy** | **- thy** | **+ thv** | | **TC** | **Spores** | **%** |
| PY₇₉ | *thyA*⁺ *thyB*⁺ | + | + | + | + | 0.25 | 2.8 × 10⁸ | 2.4 ×10⁸ | 85.7 |
| SH11 | *thyA::cotC-vp26* | + | + | - | + | 16 | 1.9 × 10⁸ | 1.6 ×10⁸ | 82 |
| SH12 | *thyA::cotC-vp26 thyB::cotB-vp28* | - | + | - | + | >64 | 2 × 10⁸ | 1.5 × 10⁸ | 75 |
| AC01 | *thyA::cotB-vp28* | + | + | - | + | 16 | 2.6 × 10⁸ | 2.2 × 10⁸ | 85 |
| AC02 | *thyA::cotB-vp28 thyB::cotB-vp28* | - | + | - | + | >64 | 1.9 ×10⁸ | 1.5 × 10⁸ | 78.6 |
| SH13 | *thyA::cotB-tcdA₂₆₋₃₉* | + | + | - | + | 16 | 2.2 ×10^{$} | 1.8 × 10⁸ | 85 |
| SH14 | *thyA::cotB-tcdA₂₆₋₃₉ thyB::cotC-tcdA₂₆₋₃₉* | - | + | - | + | >64 | 2.5 ×10⁸ | 1.9 ×10⁸ | 77.2 |
| SH15 | *thyA::cotB-SA* | + | + | - | + | 16 | 2.8 × 10⁸ | 2.3 × 10⁸ | 82 |
| SH16 | *thyA::cotB-SA thyB::cotB-SA* | - | + | - | + | >64 | 3.1 × 10⁸ | 2.4 × 10⁸ | 77.4 |
| SH17 | *thyA::cotB-amyE* | + | + | - | + | | | | |
| SH18 | *thyA:: co tB-amyE thyB::MCS* | - | + | - | + | | | | |
| SH19 | *thyA::cotB-aprE* | + | + | - | + | 16 | 8.7 × 10⁸ | 5.5 × 10⁸ | 90 |
| SH20 | *thyA::cotB-aprE thyB::MCS* | - | + | - | + | >64 | 8.1 × 10⁷ | 6.8 × 10⁷ | 83.9 |

| | | | | | | | | | |
|---|---|---|---|---|---|---|---|---|---|
| ¹ growth (+) or no growth (-) on SMM agar with or without thymine (50 µg/ml) ² minimal inhibitory concentration (MIC; µg/ml) of trimethoprim (Tm) determined using a microdilution method (52). ³ % of heat-resistant spores in cultures grown overnight in DSM medium (+ thymine) at 37°C. TC, total count cfu/ml; Spores, heat-resistant spore count cfu/ml. | | | | | | | | | |

Finally, using this cloning system the inventors were able to introduce insertions that were of the same or opposed transcriptional polarity to the respective thy gene **(****Fig. 9 to Fig. 13****)** showing no evidence of read through from the proximal thy coding sequences.

### Example 2

### Experimental considerations for use of ectopic insertion at the thy loci

### a) Growth in rich media

In rich medium such as LB and DSM the single *thyA* insertion strain was able to grow at 37°C without thymine supplementation while the *thyA thyB* double insertion showed no growth however (**Fig. 5B** and **D**). Intriguingly, supplementing LB or DSM with thymine did not restore normal levels of growth. In LB the double insertion failed to grow (**Fig. 5A**) while in DSM plus thymine limited growth was observed (**Fig. 5C**). However, after 24h growth in DSM sporulation was shown to have occurred and the numbers of heat-resistant spores was essentially equivalent to PY₇₉ (**Table 1**).

Since the *thyA thyB* insertion strain could grow, albeit with reduced fitness, in minimal medium supplemented with thymine (see **Fig. 2C**) the inventors reasoned that one or more components of the rich medium might inhibit growth potentially by interfering with the folate pathway. A candidate was yeast extract (YE) that is present in LB and DSM medium at 5 mg/ml and 2 mg/ml respectively and absent in SMM medium. YE has been shown to inhibit thymine mutants *of E. coli* strains where the active bactericidal ingredient has been identified as adenosine (40). A second possibility was p-aminobenzoic acid (41), a component of YE and directly involved in the folate pathway (**Fig. 14**) but also bactericidal to *E. coli* (42).

The inventors grew strains (*thy*⁺ and *thyA thyB)* in LB + thymine with varying levels of YE and found that YE concentrations of ≥2 mg/ml inhibited growth of strains carrying two *thy* (*thyA* + *thyB)* insertions (**Fig. 17**). The inhibitory activity of YE could therefore explain why in LB medium the *thyA thyB* double insertion strain failed to grow in the presence of thymine (**Fig. 5A**). Similarly, in DSM where YE is present at a lower concentration reduced growth was observed (**Fig. 5C**). Finally, the inhibitory action of YE required the need for a modified growth medium (SOC2) lacking YE for preparation of cells for the electroporation step used in the strain constructions (see Methods).

Surprisingly, although YE clearly inhibits, the inventors have been unable to attribute this definitively to either adenosine or p-amnobenzoic acid (data not shown).

### b) Gene transfer

As mentioned earlier DNA-mediated transformation of competent cells could be used to introduce plasmid DNA into *B. subtilis* cells. However, for introducing DNA into *thyA* cells in the 2^{nd} step, electroporation using a modified medium lacking YE yielded higher frequencies of integration.

The inventors found that following the 1^{st} genetic cross two colony types, in equal proportion, were apparent on SMM agar supplemented with thymine and trimethoprim (3 µg/ml) (**Fig. 18**). Type 1 were large opaque colonies (2-3mm) and Type 2 colonies were translucent, smaller (1mm) and grew slowly. All colonies could grow at 46°C in the presence of thymine indicating *prima facie* a *thyA* insertion. However, only about one third of type 1 colonies were found using PCR to carry stable *thyA* insertions while no type 2 colonies carried insertions. The inventors assume that these colonies able to grow on trimethoprim plates must carry some form of compensatory, yet unstable, mutation/s allowing growth in the presence of the antibiotic.

A second important finding was that for the 2^{nd} genetic transfer recombinants could only be selected on SMM minimal media supplemented with thymine, trimethoprim (6 µg/ml) and CAA. If plated directly onto agar lacking CAA small (<1mm), slow growing colonies would result but after reculture these were found to have lost the *thyB* insertion as determined by colony PCR. Even in the presence of CAA all colonies were small and only about 20% of those growing on trimethoprim (6 µg/ml) carried a stable *thyB* insertion. Work in *E. coli* as well as *B. subtilis* has shown that disruption of the folate pathway can lead to depletion of key amino acids as well as purines and pyrimidines (43, 44). Trimethoprim-mediated inactivation of DHFR would deplete intracellular levels of THF, MTHF as well as DHF. In turn this would affect the reversible interconversion of serine and glycine with tetrahydrofolic acid (THF), a vital reaction in the synthesis of purines and catalysed by a serine hydroxymethyltransferase (SHMT or GlyA, (45, 46)). MTHF is also utilised in the final step of the biosynthetic pathways of cysteine and methionine (47) and disruption of the pathway by the *thyA thyB* alleles could introduce a requirement for methionine.

The inventors measured the growth of a *thyA thyB* insertion strain SH14 in SMM supplemented with CAA, adenine and thymine (**Fig. 19**). In the presence of trimethoprim (6 µg/ml) growth was optimal reaching a maximal OD₆₀₀ of ~2 only in SMM containing CAA while in media containing no CAA or carrying only adenine and/or thymine growth was markedly reduced. In the absence of trimethoprim growth of SH14 remained weak compared to the wild type strain PY₇₉ but was i) superior to that in the presence of the antibiotic, ii) growth was restored to normal fitness only in the presence of CAA. Trimethoprim therefore disrupts the folate pathway significantly reducing strain fitness and this could not be restored by supplementation with purines or pyrimidines but only with CAA.

Therefore, for the second genetic transfer step (i.e., for construction of the double, *thyA thyB,* insertion) selective media may preferably provide all amino acids. However, once constructed the use of trimethoprim is no longer required and strains can be cultivated on any media so long as three criteria are met, first, that the media contains thymine or thymidine, second, YE is either absent or at a concentration less than 2 mg/ml, and third, amino acids are provided in the growth medium.

### c) Choice of one coat protein anchor

For expression of heterologous proteins on the spore surface the coat proteins CotB and CotC can be used for both mono or divalent expression. As expected, when using two different spore coat anchors expression of a protein was higher than using expression from only one. So, as shown in **Fig. 4A**, TcdA₂₆₋₃₉ levels were higher in SH14 spores carrying *thyA::cotB-tcdA₂₆₋₃₉* and *thyB::cotC-tcdA₂₆₋₃₉* insertions than in SH13 spores carrying only a *thyA::cotB-tcdA₂₆₋₃₉* insertion. Interestingly, using fusion of VP28 to CotB and insertion of this chimera at the *thyA* locus alone (strain AC01) or at both the *thyA* and *thyB* loci (strain AC02) lead to higher levels of expression in the latter. This finding requires some consideration since it must be assumed that each spore would carry a defined number of CotE monomers that could assemble onto the spore surface and simply increasing the number should not lead to higher levels of incorporation in the coat. These strains would however, carry an intact *cotB* gene (residing at its normal chromosomal locus) so in cells carrying a *thyA::cotB-VP28* insertion (i.e., AC01) 50% of displayed CotB proteins should present a wild type CotB and 50% CotB-VP28. In a double *thyA thyB* insertion strain (AC02) the inventors would predict ~ 66% of displayed CotB proteins would present VP28 and ~33% wild type CotB. This stoichiometry ratio of CotB-VP28 and CotB would agree with the ELISA detection of VP28 as shown in **Fig. 4B****.**

### Example 3

### Reversion

An insertion generated by a double-crossover recombinational event should be inherently stable yet there exists the possibility of acquisition of a compensatory suppressor or bypass mutation. To address this the inventors conducted an experiment to determine whether upon repeated culture in the absence of any selective pressure the thymine dependence could be lost. As shown in **Table 2** repeated culture and reculture of SH14 *(thyA: :cotB-tcdA₂₆₋₃₉ thyA::cotC-tcdA₂₆₋₃₉*) yielded no loss of the thymine dependency showing the insertions were stable and suggesting that the acquisition of compensatory mutations, if they were to occur, must be an extremely rare event.

**Table 2: Reversion¹**

| **Culture** | **CFU Culture 1** | | **CFU Culture 2** | |
|---|---|---|---|---|
| | **DSM + thymine** | **DSM** | **DSM + thymine** | **DSM** |
| 1st | 2.8 × 10⁸ | 0 | 2.1 × 10⁸ | 0 |
| 2nd | 1.8 × 10⁸ | 0 | 3.4 × 10⁸ | 0 |
| 3rd | 1.85 × 10⁸ | 0 | 1.73 × 10⁸ | 0 |
| 4th | 1.82 × 10⁸ | 0 | 1.6 × 10⁸ | 0 |
| 5th | 1.54 × 10⁸ | 0 | 2.4 × 10⁸ | 0 |

| | | | | |
|---|---|---|---|---|
| ¹ Two 25ml cultures of SH14 (*thyA::cotB-tcdA₂₆₋₃₉ thyA: :cotC-tcdA₂₆₋₃₉*) were made in DSM + thymine (50 µg/ml). The culture was grown for 24h at 37°C and a sample analyzed for CFU on either DSM agar with or without thymine (50 µg/ml). A sample was also used to subculture fresh medium (25ml) and the process repeated four times. CFU/ml for each reculture are shown. | | | | |

### Example 4

### In vivo Fate of thyA⁻ thyB⁻ Spores in the GI-tract

It has been demonstrated that for *E. coli* to colonise the murine GI-tract synthesis of purines and pyrimidines is necessary (48). This implies that the low levels of purines and pyrimidines that might result from digested food or from spurious lysis of resident gut microbiota would not be sufficient to permit growth of a *B. subtilis thyA thyB* mutant. In humans the intestinal concentration of thymidine is estimated as 0.075 µM and in pigs ~1.0 µM (49). The inventors gave mice a single oral dose of 2 × 10⁹ spores of SH250 (*thyA*⁺ *thyB⁺* Cm^{R}) or SH14 *(thyA: :cotB-tcdA₂₆₋₃₉ thyA::cotC-tcdA₂₆₋₃₉*) spores by intra-gastric gavage. Subsequent shedding of heat-resistant spores in freshly voided faeces was determined. For SH250 the strain carried a silent insertion of the *cat* gene (chloramphenicol acetyltransferase) so after serial dilution heat resistant CFU was determined on chloramphenicol plates. SH14 spores were shed in the faeces and could only be detected on agar plates supplemented with thymine but when plated on plates lacking thymine no CFU could be detected (**Fig. 6**). After 10 days the number of SH14 spores being shed in the faeces was at levels (<10³/g of faeces) at the threshold of detection. This in *vivo* data shows that the kinetics of spore shedding of *thyA⁻ thyB⁻*spores are indistinguishable from shedding of spores of a wild type strain. The inventors have confirmed that SH14 spores germinate efficiently and are equivalent to wild type spores (data not shown). Although it is not possible to definitively determine whether SH14 spores could proliferate in the GI-tract it was possible to show that *thyA thyB* spores that transited the GI-tract were unable to survive in the absence of thymine. Also, there was no evidence of reversion or acquisition of markers that might enable germinated spores to survive.

### Example 5

### Utility of Spore Display

The inventors used three examplars to demonstrate that spores carrying insertions at the *thyA* and *thyB* loci were suitable for applied purposes, as vaccine delivery vehicles, for conjugation of proteins to the spore surface and finally for display of active enzymes. As vaccines the inventors used SH14 spores that express the TcdA₂₆₋₃₉ antigen of C. *difficile* fused to two spore coat protein anchors, CotB and CotC. SH14 is equivalent to strain PP108 that has previously been shown to confer protection to C. *difficile* infection (CDI) in murine and hamster models of infection (4, 38). The inventors immunised mice with spores of SH14 and PP108 using oral administration and after a total of four doses measured TcdA₂₆₋₃₉-specific IgG (**Fig. 7A**) and IgA (**Fig. 7B**) in the serum and faeces respectively. Compared to control groups (naive and mice dosed with naked PY₇₉ spores) that exhibited no responses, both PP108 and SH14 spores generated high titres of IgG and IgA and based on previous work the inventors would predict these levels of antibodies would be protective.

For conjugation of proteins to streptavidin displayed on the spore surface the inventors first biotinylated a rabbit polyclonal TcdA₂₆₋₃₉-specific antibody and demonstrated that the antibody conjugated to SH16 spores using immunofluorescence detection (**Fig. 8A**) and also confirmed using whole spore ELISA (not shown). Using these conjugated spores the inventors asked whether SH16 spores displaying TcdA₂₆₋₃₉ IgG could subtract C. *difficile* toxins from a crude cell-free lysate. As shown in **Table 3** incubation of conjugated spores with toxin-containing lysates for just 5 min. reduced toxicity by 90%. Interestingly, PY₇₉ spores also had some ability to bind TcdA₂₆₋₃₉ antibodies and were able to provide a modest reduction (10-20%) in toxin activity. Used as an example this experiment demonstrates that spores might have potential for therapeutic purposes, for example in the oral administration of antibodies.

**Table 3: Subtraction of C. difficile toxins¹**

| **Mixture** | **Cytotoxicity (%)** |
|---|---|
| Medium only | 0 |
| Toxins only | 100 |
| PY₇₉ treated spores+ toxins | 80-90 |
| SH16 conjugated spores + toxin | 10 |

| | |
|---|---|
| ¹ Cytoxicity in HT29 cells was measured using crude toxins from C. *difficile* or from the same toxins pretreated with SH16 spores conjugated with a polyclonal antibody to TcdA₂₆₋₃₉ or PY₇₉ treated spores. | |

Enzymes are commonly included in animal feeds where they improve digestion and nutrition. Proteases and amylases are pertinent examples used here to show that it is possible to display an enzyme that retains activity on the spore surface. SH18 spores expressing amylase were found able to express active amylase on their surface (Fig. **8B**). Using casein agar we showed that SH20 spores expressing the alkaline protease, subtilisin E, carried enzymatic activity (**Fig. 8B**). In liquid suspensions we found that 10¹⁰ spores of SH20 had ~0.127 units of protease activity.

### Summary of Examples 1 to 5

The inventors have described a straightforward method to contain genetically modified bacterial spores. Their approach expands yet differs from those described for *Lactobacillus acidophilus* (51) and *Lactococcus lactis* (Steidler *et al.,* 2003) that rely on the indigenous suicide resulting from a 'thymine-less death'. First described in 1954 (23) thymine dependence differs from other auxotrophies in that the absence of thymine is bactericidal and so bacteria carrying defects in the thymidylate synthase genes cannot accumulate in the environment. *Bacillus* species carry two thymidylate synthase genes (*thyA* and *thyB)* requiring inactivation of both loci to achieve complete dependence on thymine. The inventors have shown here that this is possible and have identified a two-step cloning procedure requiring insertional inactivation of first *thyA* and then *thyB* loci. Their approach does not require the introduction of antibiotic resistance markers for selection but rather the development of increasing levels of resistance to trimethoprim that arise from successive disruption of the folate pathway. Coupled with the temperature sensitive phenotype of insertions at the *thyA* and *thyB* loci this provides a useful method for both selection and screening of insertion although technically there are a number of constraints that must be considered. The inventors show that the absence of thymine is bactericidal and observe no evidence for reversion or suppression despite repeated passage of these strains. Of course, the purpose of the cloning system is to construct strains able to express proteins for applied purposes, for example, for expression of heterologous antigens or enzymes. The inventors have used examples here showing that chimeric proteins comprised of a foreign protein fused to a spore coat protein can be displayed on the spore surface. This has included the delivery of two enzymes (subtilisin E and α-amylase), putative vaccine protective antigens as well as streptavidin which was used to conjugate to a polyclonal antibody. It is clear though that this system could equally be used for expression of proteins in, or secretion from, the vegetative cell.

### References

1. Nicholson WJ, Munakata N, Horneck G, Melosh HJ, Setlow P. 2000. Resistance of Bacillus endospores to extreme terrestial and extraterrestrial environments. Microbiology and Molecular Biology Reviews 64:548-572.
2. Pan JG, Kim EJ, Yun CH. 2012. Bacillus spore display. Trends Biotechnol 30:610-612.
3. Due LH, Hong HA, Fairweather N, Ricca E, Cutting SM. 2003. Bacterial spores as vaccine vehicles. Infection and Immunity 71:2810-2818.
4. Permpoonpattana P, Hong HA, Phetcharaburanin J, Huang JM, Cook J, Fairweather NF, Cutting SM. 2011. Immunization with Bacillus spores expressing toxin A peptide repeats protects against infection with Clostridium difficile strains producing toxins A and B. Infection and immunity 79:2295-2302.
5. Lee S, Belitsky BR, Brinker JP, Kerstein KO, Brown DW, Clements JD, Keusch GT, Tzipori S, Sonenshein AL, Herrmann JE. 2010. Development of a Bacillus subtilis-based rotavirus vaccine. Clin Vaccine Immunol 17:1647-1655.
6. Fu LL, Li WF, Du HH, Dai W, Xu ZR. 2008. Oral vaccination with envelope protein VP28 against white spot syndrome virus in Procambarus clarkii using Bacillus subtilis as delivery vehicles. Lett Appl Microbiol 46:581-586.
7. Nguyen AT, Pham CK, Pham HT, Pham HL, Nguyen AH, Dang LT, Huynh HA, Cutting SM, Phan TN. 2014. Bacillus subtilis spores expressing the VP28 antigen: a potential oral treatment to protect Litopenaeus vannamei against white spot syndrome. FEMS Microbiol Lett 358:202-208.
8. Ning D, Leng X, Li Q, Xu W. 2011. Surface-displayed VP28 on Bacillus subtilis spores induce protection against white spot syndrome virus in crayfish by oral administration. Journal of applied microbiology 111:1327-1336.
9. Valdez A, Yepiz-Plascencia G, Ricca E, Olmos J. 2014. First Litopenaeus vannamei WSSV 100% oral vaccination protection using CotC::Vp26 fusion protein displayed on Bacillus subtilis spores surface. J Appl Microbiol doi:10.1111/jam.12550.
10. EFSA. 2008. The maintenance of the list of QPS microorganisms intentionally added to food or feed. EFSA Journal 923:1-48.
11. Hong HA, Due le H, Cutting SM. 2005. The use of bacterial spore formers as probiotics. FEMS Microbiol Rev 29:813-835.
12. Amalaradjou MA, Bhunia AK. 2013. Bioengineered probiotics, a strategic approach to control enteric infections. Bioengineered 4:379-387.
13. Sorokulova IB, Beliavskaia VA, Masycheva VA, Smirnov VV. 1997. Recombinant probiotics: problems and prospects of their use for medicine and veterinary practice. Vestn Ross Akad Med Nauk:46-49.
14. Potot S, Serra CR, Henriques AO, Schyns G. 2010. Display of recombinant proteins on Bacillus subtilis spores, using a coat-associated enzyme as the carrier. Appl Environ Microbiol 76:5926-5933.
15. Nguyen VA, Huynh HA, Hoang TV, Ninh NT, Pham AT, Nguyen HA, Phan TN, Cutting SM. 2013. Killed Bacillus subtilis spores expressing streptavidin: a novel carrier of drugs to target cancer cells. J Drug Target 21:528-541.
16. Shimotsu H, Henner DJ. 1986. Construction of a single copy integration vector and its use in analysis of regulation of the trp operon of Bacillus subtilis. Gene 43:85-94.
17. Guerout-Fleury AM, Frandsen N, Stragier P. 1996. Plasmids for ectopic integration in Bacillus subtilis. Gene 180:57-61.
18. Bloor AE, Cranenburgh RM. 2006. An efficient method of selectable marker gene excision by Xer recombination for gene replacement in bacterial chromosomes. Appl Environ Microbiol 72:2520-2525.
19. Iwanicki A, Piatek I, Stasilojc M, Grela A, Lega T, Obuchowski M, Hinc K. 2014. A system of vectors for Bacillus subtilis spore surface display. Microb Cell Fact 13:30.
20. Cano RJ, Borucki M. 1995. Revival and identification of bacterial spores in 25- to 40-million-year-old Dominican amber. Science 268:1060-1064.
21. Nicholson WL. 2002. Roles of Bacillus endospores in the environment. Cellular and Molecular Life Sciences 59:410-416.
22. Mauriello EM, Cangiano G, Maurano F, Saggese V, De Felice M, Rossi M, Ricca E. 2007. Germination-independent induction of cellular immune response by Bacillus subtilis spores displaying the C fragment of the tetanus toxin. Vaccine 25:788-793.
23. Cohen SS, Barner HD. 1954. Studies on Unbalanced Growth in Escherichia coli. Proc Natl Acad Sci U S A 40:885-893.
24. Goulian M, Bleile BM, Dickey LM, Grafstrom RH, Ingraham HA, Neynaber SA, Peterson MS, Tseng BY. 1986. Mechanism of thymineless death. Adv Exp Med Biol 195 Pt B:89-95.
25. Ahmad SI, Kirk SH, Eisenstark A. 1998. Thymine metabolism and thymineless death in prokaryotes and eukaryotes. Annu Rev Microbiol 52:591-625.
26. Rolfe R. 1967. On the mechanism of thymineless death in Bacillus subtilis. Proc Natl Acad Sci U S A 57:114-121.
27. Neuhard J, Price AR, Schack L, Thomassen E. 1978. Two thymidylate synthetases in Bacillus subtilis. Proc Natl Acad Sci U S A 75:1194-1198.
28. Zeigler DR, Pragai Z, Rodriguez S, Chevreux B, Muffler A, Albert T, Bai R, Wyss M, Perkins JB. 2008. The Origins of 168, W23, and Other Bacillus subtilis Legacy Strains. Journal of Bacteriology 190:6983-6995.
29. Harwood CR, Cutting SM. 1990. Molecular Biological Methods for Bacillus. John Wiley & Sons Ltd., Chichester, England.
30. Nicholson WL, Setlow P. 1990. Sporulation, germination and outgrowth., p 391-450. In Harwood. CR, Cutting. SM (ed), Molecular Biological Methods for Bacillus. John Wiley & Sons Ltd., Chichester, UK.
31. Ikemura H, Takagi H, Inouye M. 1987. Requirement of pro-sequence for the production of active subtilisin E in Escherichia coli. J Biol Chem 262:7859-7864.
32. Tran TT, Mamo G, Mattiasson B, Hatti-Kaul R. 2010. A thermostable phytase from Bacillus sp. MD2: cloning, expression and high-level production in Escherichia coli. J Ind Microbiol Biotechnol 37:279-287.
33. Dubnau D, Davidoff-Abelson R. 1971. Fate of transforming DNA following uptake by competent Bacillus subtilis. I. Formation and properties of the donor-recipient complex. Journal of Molecular Biology 56:209-221.
34. Cutting SM, Vander-Horn PB. 1990. Genetic Analysis, p 27-74. In Harwood CR, Cutting SM (ed), Molecular Biological Methods for Bacillus. John Wiley & Sons Ltd., Chichester, England.
35. Sun QY, Ding LW, He LL, Sun YB, Shao JL, Luo M, Xu ZF. 2009. Culture of Escherichia coli in SOC medium improves the cloning efficiency of toxic protein genes. Anal Biochem 394:144-146.
36. Permpoonpattana P, Phetcharaburanin J, Mikelsone A, Dembek M, Tan S, Brisson MC, La Ragione R, Brisson AR, Fairweather N, Hong HA, Cutting SM. 2013. Functional Characterization of Clostridium difficile Spore Coat Proteins. J Bacteriol 195:1492-1503.
37. Harwood CR, Coxon RD, Hancock IC. 1990. The Bacillus cell envelope and secretion, p 327-390. In Harwood CR, Cutting SM (ed), Molecular Biological Methods for Bacillus. John Wiley & Sons, Chichester.
38. Hong HA, Hitri K, Hosseini S, Kotowicz N, Bryan D, Mawas F, Wilkinson AJ, van Broekhoven A, Kearsey J, Cutting SM. 2017. Mucosal Antibodies to the C-terminus of Toxin A Prevent Colonization of Clostridium difficile Infection and Immunity 85.
39. Walsh GA, Power RF, Headon DR. 1993. Enzymes in the animal-feed industry. Trends Biotechnol 11:424-430.
40. Kinoshita N, Akiyoshi H, Endo H. 1969. Isolation and characterization of substance in yeast extract which inhibits growth of thymine-less strains of Escherichia coli. J Gen Microbiol 59:393-400.
41. Blanchard KC. 1941. The isolation of p-aminobenzoic acid from yeast. The Journal of Biological Chemistry 140:919-926.
42. Davis BD. 1951. Inhibition of Escherichia coli by p-aminobenzoic acid and its reversal by p-hydroxybenzoic acid. J Exp Med 94:243-254.
43. Amyes SG, Smith JT. 1974. Trimethoprim action and its analogy with thymine starvation. Antimicrob Agents Chemother 5:169-178.
44. Stepanek JJ, Schakermann S, Wenzel M, Prochnow P, Bandow JE. 2016. Purine biosynthesis is the bottleneck in trimethoprim-treated Bacillus subtilis. Proteomics Clin Appl 10:1036-1048.
45. Schirch L. 1982. Serine hydroxymethyltransferase. Adv Enzymol Relat Areas Mol Biol 53:83-112.
46. Ponce-de-Leon MM, Pizer LI. 1972. Serine biosynthesis and its regulation in Bacillus subtilis. J Bacteriol 110:895-904.
47. Ferla MP, Patrick WM. 2014. Bacterial methionine biosynthesis. Microbiology 160:1571-1584.
48. Vogel-Scheel J, Alpert C, Engst W, Loh G, Blaut M. 2010. Requirement of purine and pyrimidine synthesis for colonization of the mouse intestine by Escherichia coli. Appl Environ Microbiol 76:5181-5187.
49. Steidler L, Neirynck S, Huyghebaert N, Snoeck V, Vermeire A, Goddeeris B, Cox E, Remon JP, Remaut E. 2003. Biological containment of genetically modified Lactococcus lactis for intestinal delivery of human interleukin 10. Nat Biotechnol 21:785-789.
50. Yan S, Wu G. 2017. Bottleneck in secretion of alpha-amylase in Bacillus subtilis. Microb Cell Fact 16:124.
51. Fu X, Xu JG. 2000. Development of a chromosome-plasmid balanced lethal system for Lactobacillus acidophilus with thyA gene as selective marker. Microbiol Immunol 44:551-556.
52. CLSI. 2010. Methods for antimicrobial dilution and disk susceptibility testing of infrequently isolated or fastidious bacteria; approved guideline - Second Ed M45-A2. Institute CaLS, USA.
53. Barra-Carrasco, J., V. Olguin-Araneda, A. Plaza-Garrido, C. Miranda-Cardenas, G. Cofre-Araneda, M. Pizarro-Guajardo, M.R. Sarker & D. Paredes-Sabja, (2013) The Clostridium difficile exosporium cysteine (CdeC)-rich protein is required for exosporium morphogenesis and coat assembly. J Bacteriol 195: 3863-3875.
54. Permpoonpattana, P., E.H. Tolls, R. Nadem, S. Tan, A. Brisson & S.M. Cutting, (2011b) Surface layers of Clostridium difficile endospores. J Bacteriol 193: 6461-6470.
55. Phetcharaburanin, J., H.A. Hong, C. Colenutt, I. Bianconi, L. Sempere, P. Permpoonpattana, K. Smith, M. Dembek, S. Tan, M.C. Brisson, A.R. Brisson, N.F. Fairweather & S.M. Cutting, (2014) The spore-associated protein BclA1 affects the susceptibility of animals to colonization and infection by Clostridium difficile. Mol Microbiol 92: 1025-1038.

## Claims

1. A process for introducing at least two heterologous genes into a *Bacillus Spp* and rendering the *Bacillus Spp* unable to proliferate in the absence of thymine or thymidine, the process comprising:
(a)
(i) introducing a heterologous gene into the *thyA* gene and the *thyB* gene in a *Bacillus Spp;* and
(ii) growing said *Bacillus Spp* in the presence of trimethoprim; or
(b)
(i) introducing a heterologous gene into either the *thyA* gene or the *thyB* gene in a *Bacillus Spp;*
ii) growing said *Bacillus Spp* in the presence of trimethoprim;
iii) introducing a heterologous gene into the other of the *thyA* gene or the *thyB* gene in said *Bacillus Spp.;* and
(iv) growing said *Bacillus Spp.* in the presence of trimethoprim,
wherein the process operates without the introduction of an antibiotic resistance gene.

2. The process of claim 1, wherein the *Bacillus Spp.,* is *B. subtilis.*

3. The process of claim 1(b), wherein:
step (i) is the introduction of a heterologous gene into the *thyA* gene in a *Bacillus Spp.;* and
step (iii) is the introduction of a heterologous gene into the *thyB* gene in a *Bacillus Spp.*

4. The process of any one of claims 1 to 3, wherein:
during step (ii), the *Bacillus Sppis* grown in a medium comprising yeast extract (YE) at a concentration lower than 2 mg/ml, 1.5 mg/ml, 1 mg/ml, 0.5 mg/ml, 0.25 mg/ml, 0.125 mg/ml, 0.1 mg/ml, 0.01 mg/ml, 0.001 mg/ml, 0.0001 mg/ml, or 0 mg/ml, preferably the medium in which the *Bacillus Spp* is grown contains no YE.

5. The process of any one of claims 1(b), 2 or 3, wherein:
during step (iv), the *Bacillus Spp.* is grown in a medium comprising yeast extract (YE) at a concentration lower than 2 mg/ml, 1.5 mg/ml, 1 mg/ml, 0.5 mg/ml, 0.25 mg/ml, 0.125 mg/ml, 0.1 mg/ml, 0.01 mg/ml, 0.001 mg/ml, 0.0001 mg/ml, or 0 mg/ml, preferably the medium in which the *Bacillus Spp.* is grown contains no YE.

6. The process of any preceding claim, wherein:
during step (ii), the *Bacillus Spp* is grown in a medium comprising thymine or thymidine; and/or
wherein YE is present at a concentration lower than 2 mg/ml, or preferably not present, in the media used at any stage of the process.

7. The process of any one of claims 1(b), 2, 3 or 5, wherein:
during step (iv), the *Bacillus Spp.* is grown in a medium comprising thymine or thymidine.

8. The process of any one of claims 1(b), 2, 3, 5 or 7, wherein:
the amount of trimethoprim present in step (iv) is higher than the amount of trimethoprim present in step (ii).

9. The process of claim 3, wherein cells carrying an insertion at the *thyA* locus are verified by the ability to grow at 37°C with or without thymine or thymidine but the inability to grow at 46°C unless supplemented with thymine or thymidine.

10. The process of any one of claims 1(b), 2, 3, 5, 7 or 8, wherein:
during step (iv), the *Bacillus Spp.* is grown in a medium supplemented with at least one amino acid, optionally wherein:
during step (iv), the *Bacillus Spp.* is grown in a medium comprising casamino acids (CAA).

11. The process of any preceding claim:
(i) wherein cells carrying two insertions are verified by the inability to grow at 37°C or 46°C unless supplemented with thymine or thymidine;
(ii) wherein the process operates without the introduction of an antibiotic resistance gene; and/or
(iii) further comprising forming a bacterial spore from said spore-forming bacterium.

12. A bacterial spore obtained or obtainable by the process of any one of claims 1-11.

13. A bacterial spore comprising at least two heterologous genes and unable to proliferate in the absence of thymine or thymidine, wherein the bacterial spore is produced by a process comprising:
(i) introducing a heterologous gene into the *thyA* gene and the *thyB* gene in a *Bacillus Spp ;*
(ii) growing said *Bacillus Spp* in the presence of trimethoprim; and
(iii) forming a bacterial spore from said *Bacillus Spp,*
wherein the bacterial spore does not comprise an antibiotic resistance gene.

14. A bacterial spore according to claim 13, wherein the bacterial spore is produced by the process according to any one of claims 1-11.

15. A vector for use in the process of any one of claims 1-11, wherein the vector comprises at least a portion of the 5' portion of a *Bacillus Spp. thyA* gene, a heterologous gene, and at least a portion of the 3' portion of a *Bacillus Spp. thyA* gene, and further comprising at least a portion of the 5' portion of a *Bacillus Spp. thyB* gene, a heterologous gene, and at least a portion of the 3' portion of a *Bacillus Spp. thyB* gene.

## Patentansprüche

1. Verfahren zum Einführen von mindestens zwei heterologen Genen in einen *Bacillus spp.* und Unfähigmachen des *Bacillus spp.,* sich in Abwesenheit von Thymin oder Thymidin zu vermehren, wobei das Verfahren Folgendes umfasst:
(a)
(i) Einführen eines heterologen Gens in das *thyA-Gen* und das *thyB-Gen* in einem *Bacillus spp.*; und
(ii) Züchten des *Bacillus spp.* bei Anwesenheit von Trimethoprim; oder
(b)
(i) Einführen eines heterologen Gens entweder in das *thyA-Gen* oder das *thyB-Gen* in einem *Bacillus spp.,*
ii) Züchten des *Bacillus spp.* bei Anwesenheit von Trimethoprim;
iii) Einführen eines heterologen Gens in das andere von dem *thyA-*Gen oder dem *thyB-Gen* in dem *Bacillus spp.;* und
(iv) Züchten des *Bacillus spp.* bei Anwesenheit von Trimethoprim, wobei das Verfahren ohne die Einführung eines Antibiotikaresistenzgens abläuft.

2. Verfahren nach Anspruch 1, wobei der *Bacillus spp., B. subtilis* ist.

3. Verfahren nach Anspruch 1(b), wobei:
Schritt (i) die Einführung eines heterologen Gens in das *thyA-Gen* in einen *Bacillus spp.* ist; und
Schritt (iii) die Einführung eines heterologen Gens in das *thyB-Gen* in einem *Bacillus spp.* ist.

4. Verfahren nach einem der Ansprüche 1 bis 3, wobei:
während des Schritts (ii) der *Bacillus spp.* in einem Medium gezüchtet wird, das Hefeextrakt (YE) in einer Konzentration von weniger als 2 mg/ml, 1,5 mg/ml, 1 mg/ml, 0,5 mg/ml, 0,25 mg/ml, 0,125 mg/ml, 0,1 mg/ml, 0,01 mg/ml, 0,001 mg/ml, 0,0001 mg/ml oder 0 mg/ml umfasst, wobei das Medium, in dem der *Bacillus spp.* gezüchtet wird, vorzugsweise kein YE enthält.

5. Verfahren nach einem der Ansprüche 1(b), 2 oder 3, wobei:
während des Schritts (iv) der *Bacillus spp.* in einem Medium gezüchtet wird, das Hefeextrakt (YE) in einer Konzentration von weniger als 2 mg/ml, 1,5 mg/ml, 1 mg/ml, 0,5 mg/ml, 0,25 mg/ml, 0,125 mg/ml, 0,1 mg/ml, 0,01 mg/ml, 0,001 mg/ml, 0,0001 mg/ml oder 0 mg/ml umfasst, wobei das Medium, in dem der *Bacillus spp.* gezüchtet wird, vorzugsweise kein YE enthält.

6. Verfahren nach einem vorhergehenden Anspruch, wobei:
während des Schritts (ii) der *Bacillus spp.* in einem Medium gezüchtet wird, das Thymin oder Thymidin umfasst; und/oder
wobei YE in einer Konzentration von weniger als 2 mg/ml vorhanden ist oder vorzugsweise nicht in den Medien vorhanden ist, die in einem beliebigen Stadium des Verfahrens verwendet werden.

7. Verfahren nach einem der Ansprüche 1(b), 2, 3 oder 5, wobei:
während des Schritts (iv) der *Bacillus spp.* in einem Medium gezüchtet wird, das Thymin oder Thymidin umfasst.

8. Verfahren nach einem der Ansprüche 1(b), 2, 3, 5 oder 7, wobei:
die Menge an Trimethoprim, die in Schritt (iv) vorhanden ist, höher als die Menge an Trimethoprim ist, die in Schritt (ii) vorhanden ist.

9. Verfahren nach Anspruch 3, wobei Zellen, die eine Einfügung an dem thyA-Locus tragen, dadurch verifiziert werden, dass sie dazu in der Lage sind, bei 37 °C mit oder ohne Thymin oder Thymidin gezüchtet zu werden, aber nicht dazu in der Lage sind, bei 46 °C gezüchtet zu werden, es sei denn, sie sind mit Thymin oder Thymidin ergänzt.

10. Verfahren nach einem der Ansprüche 1(b), 2, 3, 5, 7 oder 8, wobei:
während des Schritts (iv) der *Bacillus spp.* in einem Medium gezüchtet wird, das mit mindestens einer Aminosäure ergänzt ist, wobei optional:
während des Schritts (iv) der *Bacillus spp.* in einem Medium gezüchtet wird, das Casaminosäuren (CAA) umfasst.

11. Verfahren nach einem vorhergehenden Anspruch:
(i) wobei Zellen, die zwei Einfügungen tragen, dadurch verifiziert werden, dass sie nicht dazu in der Lage sind, bei 37 °C oder 46 °C gezüchtet zu werden, es sei denn, sie sind mit Thymin oder Thymidin ergänzt;
(ii) wobei das Verfahren ohne die Einführung eines Antibiotikaresistenzgens abläuft; und/oder
(iii) ferner umfassend Bilden einer bakteriellen Spore aus dem sporenbildenden Bakterium.

12. Bakterielle Spore, erhalten oder erhaltbar durch das Verfahren nach einem der Ansprüche 1-11.

13. Bakterielle Spore, umfassend mindestens zwei heterologe Gene, die nicht dazu in der Lage ist, sich in Abwesenheit von Thymin oder Thymidin zu vermehren, wobei die bakterielle Spore durch ein Verfahren hergestellt wird, das Folgendes umfasst:
(i) Einführen eines heterologen Gens in das *thyA-*Gen und das *thyB-Gen* in einem *Bacillus spp.;*
(ii) Züchten des *Bacillus spp.* bei Anwesenheit von Trimethoprim; und
(iii) Bilden einer bakteriellen Spore aus dem *Bacillus spp.,*
wobei die bakterielle Spore kein Antibiotikaresistenzgen umfasst.

14. Bakterielle Spore nach Anspruch 13, wobei die bakterielle Spore durch das Verfahren nach einem der Ansprüche 1-11 hergestellt wird.

15. Vektor zur Verwendung in dem Verfahren nach einem der Ansprüche 1-11, wobei der Vektor mindestens einen Abschnitt des 5'-Abschnitts eines *Bacillus-spp.-thyA-Gens,* ein heterologes Gen und mindestens einen Abschnitt des 3'-Abschnitts eines *Bacillus-spp.-thyA-Gens* umfasst, und ferner umfassend mindestens einen Abschnitt des 5'-Abschnitts eines *Bacillus-spp.-thyB-Gens,* ein heterologes Gen und mindestens einen Abschnitt des 3'-Abschnitts eines *Bacillusspp. -thyB-Gens.*

## Revendications

1. Procédé permettant d'introduire au moins deux gènes hétérologues dans un *Bacillus Spp* et de rendre le *Bacillus Spp* incapable de proliférer en l'absence de thymine ou de thymidine, le procédé comprenant :
(a)
(i) l'introduction d'un gène hétérologue dans le gène *thyA* et le gène *thyB* dans un *Bacillus Spp ;* et
(ii) la culture dudit *Bacillus Spp* en présence de triméthoprime ; ou
(b)
(i) l'introduction d'un gène hétérologue soit dans le gène *thyA* soit dans le gène *thyB* dans un *Bacillus Spp ;*
ii) la culture dudit *Bacillus Spp* en présence de triméthoprime ;
iii) l'introduction d'un gène hétérologue dans l'autre gène parmi le gène *thyA* ou le gène *thyB* dans ledit *Bacillus Spp.* ; et
iv) la culture dudit *Bacillus Spp.* en présence de triméthoprime,
ledit procédé fonctionnant sans introduction d'un gène de résistance aux antibiotiques.

2. Procédé selon la revendication 1, ledit *Bacillus Spp.* étant *B. subtilis.*

3. Procédé selon la revendication 1(b) :
ladite étape (i) étant l'introduction d'un gène hétérologue dans le gène *thyA* dans un *Bacillus Spp.* ; et
ladite étape (iii) étant l'introduction d'un gène hétérologue dans le gène *thyB* dans un *Bacillus Spp.*

4. Procédé selon l'une quelconque des revendications 1 à 3 :
lors de ladite étape (ii), ledit *Bacillus Spp* étant cultivé dans un milieu comprenant un extrait de levure (YE) à une concentration inférieure à 2 mg/ml, 1,5 mg/ml, 1 mg/ml, 0,5 mg/ml, 0,25 mg/ml, 0,125 mg/ml, 0,1 mg/ml, 0,01 mg/ml, 0,001 mg/ml, 0,0001 mg/ml ou 0 mg/ml, de préférence ledit milieu dans lequel *Bacillus Spp* est cultivé ne contenant pas de YE.

5. Procédé selon l'une quelconque des revendications 1(b), 2 ou 3 :
lors de ladite étape (iv), ledit *Bacillus Spp.* étant cultivé dans un milieu comprenant un extrait de levure (YE) à une concentration inférieure à 2 mg/ml, 1,5 mg/ml, 1 mg/ml, 0,5 mg/ml, 0,25 mg/ml, 0,125 mg/ml, 0,1 mg/ml, 0,01 mg/ml, 0,001 mg/ml, 0,0001 mg/ml ou 0 mg/ml, de préférence ledit milieu dans lequel *Bacillus Spp.* est cultivé ne contenant pas de YE.

6. Procédé selon l'une quelconque des revendications précédentes :
lors de ladite étape (ii), ledit *Bacillus Spp* étant cultivé dans un milieu comprenant de la thymine ou de la thymidine ; et/ou
ledit YE étant présent à une concentration inférieure à 2 mg/ml, ou de préférence non présent, dans les milieux utilisés à n'importe quel stade du procédé.

7. Procédé selon l'une quelconque des revendications 1(b), 2, 3 ou 5 :
lors de ladite étape (iv), ledit *Bacillus Spp.* étant cultivé dans un milieu comprenant de la thymine ou de la thymidine.

8. Procédé selon l'une quelconque des revendications 1(b), 2, 3, 5 ou 7 :
ladite quantité de triméthoprime présente à l'étape (iv) étant supérieure à la quantité de triméthoprime présente à l'étape (ii).

9. Procédé selon la revendication 3, les cellules portant une insertion au niveau du locus *thyA* étant vérifiées par la capacité de croître à 37°C avec ou sans thymine ou thymidine mais l'incapacité de croître à 46°C à moins d'être complétées par de la thymine ou de la thymidine.

10. Procédé selon l'une quelconque des revendications 1(b), 2, 3, 5, 7 ou 8 :
lors de ladite étape (iv), ledit *Bacillus Spp.* étant cultivé dans un milieu complété par au moins un acide aminé, éventuellement :
lors de ladite étape (iv), ledit *Bacillus Spp* étant cultivé dans un milieu comprenant des acides casaminés (CAA).

11. Procédé selon l'une quelconque des revendications précédentes :
(i) lesdites cellules portant deux insertions étant vérifiées par l'incapacité de croître à 37°C ou à 46°C à moins d'être complétées par de la thymine ou de la thymidine ;
(ii) ledit procédé fonctionnant sans introduction d'un gène de résistance aux antibiotiques ; et/ou
(iii) comprenant en outre la formation d'une spore bactérienne à partir de ladite bactérie sporogène.

12. Spore bactérienne obtenue ou pouvant être obtenue par le procédé selon l'une quelconque des revendications 1-11.

13. Spore bactérienne comprenant au moins deux gènes hétérologues et incapable de proliférer en l'absence de thymine ou de thymidine, ladite spore bactérienne étant produite par un procédé comprenant :
(i) l'introduction d'un gène hétérologue dans le gène *thyA* et le gène *thyB* dans un *Bacillus Spp ;*
(ii) la culture dudit *Bacillus Spp* en présence de triméthoprime ; et
(iii) la formation d'une spore bactérienne à partir dudit *Bacillus Spp,*
ladite spore bactérienne ne comprenant pas de gène de résistance aux antibiotiques.

14. Spore bactérienne selon la revendication 13, ladite spore bactérienne étant produite par le procédé selon l'une quelconque des revendications 1-11.

15. Vecteur destiné à être utilisé dans le procédé selon l'une quelconque des revendication 1-11, ledit vecteur comprenant au moins une partie de la partie 5' d'un gène *thyA* de *Bacillus Spp,* un gène hétérologue, et au moins une partie de la partie 3' d'un gène *thyA* de *Bacillus Spp,* et comprenant en outre au moins une partie de la partie 5' d'un gène *thyB* de *Bacillus Spp.,* un gène hétérologue, et au moins une partie de la partie 3' d'un gène *thyB* de *Bacillus Spp.*
